# EUROPEAN PATENT SPECIFICATION

(11) **EP 3 086 802 B1**
(45) Date of publication and mention of the grant of the patent: **19.09.2018**
(21) Application number: 14833578.9
(22) Date of filing: 22.12.2014
(51) Int. Cl.: A61K 38/16, C07K 14/705, A61P 35/00

(54) **CD44 BINDING PEPTIDES**
CD44-BINDENDE PEPTIDE
PEPTIDES SE LIANT À CD44

(30) Priority: 23.12.2013 EP 13199325
(43) Date of publication of application: 02.11.2016
(73) Proprietor: Exchange Imaging Technologies GmbH, 64287 Darmstadt (DE)
(72) Inventor: ARNTZ, Claudia, 49525 Lengerich (DE); GREB, Wolfgang, 40489 Düsseldorf (DE)
(74) Representative: Jostarndt Patentanwalts-AG
(86) International application number: PCT/EP2014/079028
(87) International publication number: WO 2015/097170

(56) References cited:
- WO-A1-95/04547
- WO-A1-2005/007700
- DATABASE GSP [Online] 18 November 2004 (2004-11-18), "Human Elk1 phosphorylation/Elk1 kinase activation protein - SEQ ID 10.", XP002725223, retrieved from EBI accession no. GSP:ADR58907 Database accession no. ADR58907 -& WO 2004/072277 A2 (ASAHI KASEI PHARMA CORP [JP]; MATSUZAKI OSAMU [JP]; MATSUDA AKIO [JP]) 26 August 2004 (2004-08-26)
- DATABASE Geneseq [Online] 9 July 2009 (2009-07-09), "Human pancreatic cancer associated target (PCAT) protein, SEQ ID:14.", XP002725224, retrieved from EBI accession no. GSP:AWV72632 Database accession no. AWV72632 -& US 2009/138977 A1 (DOMON BRUNO [US] ET AL) 28 May 2009 (2009-05-28)
- DATABASE Geneseq [Online] 13 October 2011 (2011-10-13), "Human lung cancer target LCAT protein sequence SEQ: 1.", XP002725225, retrieved from EBI accession no. GSP:AZM18611 Database accession no. AZM18611 -& US 2011/212085 A1 (JOSELOFF ELIZABETH [US] ET AL) 1 September 2011 (2011-09-01)
- SEKI KENICHIRO ET AL: "Inhibition of liver metastasis formation by anti-CD44 variant exon 9 monoclonal antibody", INTERNATIONAL JOURNAL OF ONCOLOGY, vol. 11, no. 6, 1 December 1997 (1997-12-01), pages 1257-1261, XP009124463, DEMETRIOS A. SPANDIDOS ED. & PUB, GR ISSN: 1019-6439
- Youhei Kimura ET AL: "CD44variant exon 9 plays an important role in colon cancer initiating cells", Oncotarget, 6 June 2013 (2013-06-06), pages 785-791, XP055120901, United States Retrieved from the Internet: URL:http://www.ncbi.nlm.nih.gov/pmc/articl es/PMC3742838/pdf/oncotarget-04-785.pdf [retrieved on 2014-05-30]
- HYE-YEON PARK ET AL: "Screening of Peptides Bound to Breast Cancer Stem Cell Specific Surface Marker CD44 by Phage Display", MOLECULAR BIOTECHNOLOGY ; PART B OF APPLIED BIOCHEMISTRY AND BIOTECHNOLOGY, vol. 51, no. 3, 7 October 2011 (2011-10-07), pages 212-220, XP035059773, HUMANA PRESS INC, NEW YORK ISSN: 1559-0305, DOI: 10.1007/S12033-011-9458-7
- DATABASE Geneseq [Online] 22 July 2010 (2010-07-22), "Human cell surface adhesion molecule (CD44vRA) peptide, SEQ ID 23.", XP002729839, retrieved from EBI accession no. GSP:AYB86388 Database accession no. AYB86388 -& WO 2010/058396 A1 (YISSUM RES DEV CO [IL]; NAOR DAVID [IL]; NEDVETZKI SHLOMO [IL]; GOLAN) 27 May 2010 (2010-05-27)
- DATABASE Geneseq [Online] 23 September 1998 (1998-09-23), "Human CD44 exon v5 protein sequence.", XP002729840, retrieved from EBI accession no. GSP:AAW59423 Database accession no. AAW59423 -& DE 196 48 209 A1 (BOEHRINGER INGELHEIM INT [DE]) 28 May 1998 (1998-05-28)
- AFIFY ALAA M ET AL: "Expression of CD44S and CD44v5 is more common in stage III than in stage I serous ovarian carcinomas", APPLIED IMMUNOHISTOCHEMISTRY, vol. 9, no. 4, 1 December 2001 (2001-12-01), pages 309-314, XP009178409, LIPPINCOTT WILLIAMS AND WILKINS, PHILADELPHIA, PA, US ISSN: 1062-3345
- DATABASE DAD [Online] 3 August 2008 (2008-08-03), "BAG54327.1: Homo sapiens protein (Homo sapiens cDNA FLJ44468 fis,clone UTERU2026025, moderately similar to SPLICING FACTOR,ARGININE/SERINE-RICH 2.", XP002738474, retrieved from DDBJ - DNA Data Bank of Japan Database accession no. BAG54327.1
- DATABASE Geneseq [Online] 21 June 2012 (2012-06-21), "Human derived cancer-associated target protein SEQ ID NO:673.", XP002738475, retrieved from EBI accession no. GSP:AZV55426 Database accession no. AZV55426 -& US 8 168 586 B1 (FANG DONG [US] ET AL) 1 May 2012 (2012-05-01)
- DATABASE UniProt [Online] 3 November 2009 (2009-11-03), "SubName: Full=Sodium/potassium-transporting ATPase subunit beta-3 {ECO:0000313|Ensembl:ENSP00000418353};", XP002738476, retrieved from EBI accession no. UNIPROT:C9J6S2 Database accession no. C9J6S2 & BURKARD THOMAS R ET AL: "Initial characterization of the human central proteome", BMC SYSTEMS BIOLOGY, BIOMED CENTRAL LTD, LO, vol. 5, no. 1, 26 January 2011 (2011-01-26), page 17, XP021090536, ISSN: 1752-0509, DOI: 10.1186/1752-0509-5-17
- DATABASE Geneseq [Online] 18 February 2010 (2010-02-18), "Human Na-K ATPase beta3 CAT protein SEQ ID:130.", XP002738477, retrieved from EBI accession no. GSP:AXT76926 Database accession no. AXT76926 -& US 7 582 441 B1 (RUBEN STEVE [US] ET AL) 1 September 2009 (2009-09-01)
- DATABASE UniProt [Online] 18 March 2008 (2008-03-18), "SubName: Full=ATPase Na+/K+ transporting beta 1 polypeptide {ECO:0000313|EMBL:ABS83510.1}; Flags: Fragment;", XP002738478, retrieved from EBI accession no. UNIPROT:B0LAB6 Database accession no. B0LAB6

### SEQUENCE LISTING

<110> Endosignals Medizintechnik GmbH
<120> CD44 binding peptides
<130> 12978-PT-WO
<160> 62
<170> BiSSAP 1.2
<210> 1
   <211> 32
   <212> PRT
   <213> Homo sapiens
<400> 1
<210> 2
   <211> 33
   <212> PRT
   <213> Homo sapiens
<400> 2
   Pro Gly Leu Gln Ile Ser Phe Ala Val Gln Val Pro Val Ser Val Gln
   1               5                   10                  15
   Glu Ser Ser Pro Ser Val Gln Glu Gly Ile Gln Ile Gln Val Ala Ile
               20                  25                  30
   Glu
<210> 3
   <211> 53
   <212> PRT
   <213> Homo sapiens
<400> 3
<210> 4
   <211> 66
   <212> PRT
   <213> Homo sapiens
<400> 4
   Pro Gly Pro Tyr Tyr Gly Lys Lys Leu His Val Gly Tyr Leu Gln Pro
   1               5                   10                  15
   Leu Ala Ala Val Gln Val Ser Phe Ala Pro Asn Asn Thr Gly Lys Glu
               20                  25                  30
   Val Thr Val Glu Cys Lys Ile Asp Gly Ser Ala Asn Leu Lys Ser Gln
           35                  40                  45
   Asp Asp Arg Asp Lys Phe Leu Gly Arg Val Met Phe Lys Ile Thr Ala
       50                  55                  60
   Arg Ala
   65
<210> 5
   <211> 62
   <212> PRT
   <213> Homo sapiens
<400> 5
   Tyr Tyr Pro Tyr Tyr Gly Lys Leu Leu Gln Pro Lys Tyr Leu Gln Pro
   1               5                   10                  15
   Leu Leu Ala Val Gln Phe Thr Asn Leu Thr Met Asp Thr Glu Ile Arg
               20                  25                  30
   Ile Glu Cys Lys Ala Tyr Gly Glu Asn Ile Gly Tyr Ser Glu Lys Asp
           35                  40                  45
   Arg Phe Gln Gly Arg Phe Asp Val Lys Ile Glu Val Lys Ser
       50                  55                  60
<210> 6
   <211> 38
   <212> PRT
   <213> Homo sapiens
<220>
   <223> Consensus sequence
<220>
   <221> misc_feature
   <222> (6)..(6)
   <223> Xaa can be any naturally occurring amino acid
<220>
   <221> misc_feature
   <222> (8)..(10)
   <223> Xaa can be any naturally occurring amino acid
<220>
   <221> misc_feature
   <222> (21)..(22)
   <223> Xaa can be any naturally occurring amino acid
<220>
   <221> misc_feature
   <222> (24)..(24)
   <223> Xaa can be any naturally occurring amino acid
<220>
   <221> misc_feature
   <222> (26)..(35)
   <223> Xaa can be any naturally occurring amino acid
<400> 6
   Pro Tyr Tyr Gly Lys Xaa Leu Xaa Xaa Xaa Tyr Leu Gln Pro Ser Phe
   1               5                   10                  15
   Ala Val Gln Val Xaa Xaa Ser Xaa Gln Xaa Xaa Xaa Xaa Xaa Xaa Xaa
               20                  25                  30
   Xaa Xaa Xaa Ala Ile Glu
           35
<210> 7
   <211> 39
   <212> PRT
   <213> Homo sapiens
<220>
   <223> Consensus sequence
<220>
   <221> misc_feature
   <222> (6)..(6)
   <223> Xaa can be any naturally occurring amino acid
<220>
   <221> misc_feature
   <222> (8)..(10)
   <223> Xaa can be any naturally occurring amino acid
<220>
   <221> misc_feature
   <222> (21)..(22)
   <223> Xaa can be any naturally occurring amino acid
<220>
   <221> misc_feature
   <222> (24)..(24)
   <223> Xaa can be any naturally occurring amino acid
<220>
   <221> misc_feature
   <222> (26)..(36)
   <223> Xaa can be any naturally occurring amino acid
<400> 7
   Pro Tyr Tyr Gly Lys Xaa Leu Xaa Xaa Xaa Tyr Leu Gln Pro Ser Phe
   1               5                   10                  15
   Ala Val Gln Val Xaa Xaa Ser Xaa Gln Xaa Xaa Xaa Xaa Xaa Xaa Xaa
               20                  25                  30
   Xaa Xaa Xaa Xaa Ala Ile Glu
           35
<210> 8
   <211> 40
   <212> PRT
   <213> Homo sapiens
<220>
   <223> Consensus sequence
<220>
   <221> misc_feature
   <222> (6)..(6)
   <223> Xaa can be any naturally occurring amino acid
<220>
   <221> misc_feature
   <222> (8)..(10)
   <223> Xaa can be any naturally occurring amino acid
<220>
   <221> misc_feature
   <222> (21)..(22)
   <223> Xaa can be any naturally occurring amino acid
<220>
   <221> misc_feature
   <222> (24)..(24)
   <223> Xaa can be any naturally occurring amino acid
<220>
   <221> misc_feature
   <222> (26)..(37)
   <223> Xaa can be any naturally occurring amino acid
<400> 8
   Pro Tyr Tyr Gly Lys Xaa Leu Xaa Xaa Xaa Tyr Leu Gln Pro Ser Phe
   1               5                   10                  15
   Ala Val Gln Val Xaa Xaa Ser Xaa Gln Xaa Xaa Xaa Xaa Xaa Xaa Xaa
               20                  25                  30
   Xaa Xaa Xaa Xaa Xaa Ala Ile Glu
           35                  40
<210> 9
   <211> 41
   <212> PRT
   <213> Homo sapiens
<220>
   <223> consensus sequence
<220>
   <221> misc_feature
   <222> (6)..(6)
   <223> Xaa can be any naturally occurring amino acid
<220>
   <221> misc_feature
   <222> (8)..(10)
   <223> Xaa can be any naturally occurring amino acid
<220>
   <221> misc_feature
   <222> (21)..(22)
   <223> Xaa can be any naturally occurring amino acid
<220>
   <221> misc_feature
   <222> (24)..(24)
   <223> Xaa can be any naturally occurring amino acid
<220>
   <221> misc_feature
   <222> (26)..(38)
   <223> Xaa can be any naturally occurring amino acid
<400> 9
   Pro Tyr Tyr Gly Lys Xaa Leu Xaa Xaa Xaa Tyr Leu Gln Pro Ser Phe
   1               5                   10                  15
   Ala Val Gln Val Xaa Xaa Ser Xaa Gln Xaa Xaa Xaa Xaa Xaa Xaa Xaa
               20                  25                  30
   Xaa Xaa Xaa Xaa Xaa Xaa Ala Ile Glu
           35                  40
<210> 10
   <211> 42
   <212> PRT
   <213> Homo sapiens
<220>
   <223> consensus sequence
<220>
   <221> misc_feature
   <222> (6)..(6)
   <223> Xaa can be any naturally occurring amino acid
<220>
   <221> misc_feature
   <222> (8)..(10)
   <223> Xaa can be any naturally occurring amino acid
<220>
   <221> misc_feature
   <222> (21)..(22)
   <223> Xaa can be any naturally occurring amino acid
<220>
   <221> misc_feature
   <222> (24)..(24)
   <223> Xaa can be any naturally occurring amino acid
<220>
   <221> misc_feature
   <222> (26)..(39)
   <223> Xaa can be any naturally occurring amino acid
<400> 10
   Pro Tyr Tyr Gly Lys Xaa Leu Xaa Xaa Xaa Tyr Leu Gln Pro Ser Phe
   1               5                   10                  15
   Ala Val Gln Val Xaa Xaa Ser Xaa Gln Xaa Xaa Xaa Xaa Xaa Xaa Xaa
               20                  25                  30
   Xaa Xaa Xaa Xaa Xaa Xaa Xaa Ala Ile Glu
           35                  40
<210> 11
   <211> 12
   <212> PRT
   <213> Homo sapiens
<400> 11
   Asp Val Asp Arg Asn Gly Thr Thr Ala Tyr Glu Gly
   1               5                   10
<210> 12
   <211> 12
   <212> PRT
   <213> Homo sapiens
<400> 12
   Val Asp Arg Asn Gly Thr Thr Ala Tyr Glu Gly Asn
   1               5                   10
<210> 13
   <211> 12
   <212> PRT
   <213> Homo sapiens
<400> 13
   Asp Arg Asn Gly Thr Thr Ala Tyr Glu Gly Asn Trp
   1               5                   10
<210> 14
   <211> 12
   <212> PRT
   <213> Homo sapiens
<400> 14
   Arg Asn Gly Thr Thr Ala Tyr Glu Gly Asn Trp Asn
   1               5                   10
<210> 15
   <211> 12
   <212> PRT
   <213> Homo sapiens
<400> 15
   Asn Gly Thr Thr Ala Tyr Glu Gly Asn Trp Asn Pro
   1               5                   10
<210> 16
   <211> 12
   <212> PRT
   <213> Homo sapiens
<400> 16
   Gly Thr Thr Ala Tyr Glu Gly Asn Trp Asn Pro Glu
   1               5                   10
<210> 17
   <211> 12
   <212> PRT
   <213> Homo sapiens
<400> 17
   Thr Thr Ala Tyr Glu Gly Asn Trp Asn Pro Glu Ala
   1               5                   10
<210> 18
   <211> 12
   <212> PRT
   <213> Homo sapiens
<400> 18
   Thr Ala Tyr Glu Gly Asn Trp Asn Pro Glu Ala His
   1               5                   10
<210> 19
   <211> 12
   <212> PRT
   <213> Homo sapiens
<400> 19
   Ala Tyr Glu Gly Asn Trp Asn Pro Glu Ala His Pro
   1 5 10
<210> 20
   <211> 12
   <212> PRT
   <213> Homo sapiens
<400> 20
   Tyr Glu Gly Asn Trp Asn Pro Glu Ala His Pro Pro
   1 5 10
<210> 21
   <211> 12
   <212> PRT
   <213> Homo sapiens
<400> 21
   Glu Gly Asn Trp Asn Pro Glu Ala His Pro Pro Leu
   1 5 10
<210> 22
   <211> 12
   <212> PRT
   <213> Homo sapiens
<400> 22
   Gly Asn Trp Asn Pro Glu Ala His Pro Pro Leu Ile
   1 5 10
<210> 23
   <211> 12
   <212> PRT
   <213> Homo sapiens
<400> 23
   Asn Trp Asn Pro Glu Ala His Pro Pro Leu Ile His
   1 5 10
<210> 24
   <211> 12
   <212> PRT
   <213> Homo sapiens
<400> 24
   Trp Asn Pro Glu Ala His Pro Pro Leu Ile His His
   1 5 10
<210> 25
   <211> 12
   <212> PRT
   <213> Homo sapiens
<400> 25
   Asn Pro Glu Ala His Pro Pro Leu Ile His His Glu
   1               5                   10
<210> 26
   <211> 12
   <212> PRT
   <213> Homo sapiens
<400> 26
   Pro Glu Ala His Pro Pro Leu Ile His His Glu His
   1               5                   10
<210> 27
   <211> 12
   <212> PRT
   <213> Homo sapiens
<400> 27
   Glu Ala His Pro Pro Leu Ile His His Glu His His
   1               5                   10
<210> 28
   <211> 12
   <212> PRT
   <213> Homo sapiens
<400> 28
   Ala His Pro Pro Leu Ile His His Glu His His Glu
   1               5                   10
<210> 29
   <211> 12
   <212> PRT
   <213> Homo sapiens
<400> 29
   His Pro Pro Leu Ile His His Glu His His Glu Glu
   1               5                   10
<210> 30
   <211> 12
   <212> PRT
   <213> Homo sapiens
<400> 30
   Pro Pro Leu Ile His His Glu His His Glu Glu Glu
   1               5                   10
<210> 31
   <211> 12
   <212> PRT
   <213> Homo sapiens
<400> 31
   Pro Leu Ile His His Glu His His Glu Glu Glu Glu
       1                 5                    10
<210> 32
   <211> 12
   <212> PRT
   <213> Homo sapiens
<400> 32
   Leu Ile His His Glu His His Glu Glu Glu Glu Thr
   1               5                   10
<210> 33
   <211> 12
   <212> PRT
   <213> Homo sapiens
<400> 33
   Ile His His Glu His His Glu Glu Glu Glu Thr Pro
   1               5                   10
<210> 34
   <211> 12
   <212> PRT
   <213> Homo sapiens
<400> 34
   His His Glu His His Glu Glu Glu Glu Thr Pro His
   1               5                   10
<210> 35
   <211> 12
   <212> PRT
   <213> Homo sapiens
<400> 35
   His Glu His His Glu Glu Glu Glu Thr Pro His Ser
   1               5                   10
<210> 36
   <211> 12
   <212> PRT
   <213> Homo sapiens
<400> 36
   Glu His His Glu Glu Glu Glu Thr Pro His Ser Thr
   1               5                   10
<210> 37
   <211> 12
   <212> PRT
   <213> Homo sapiens
<400> 37
   His His Glu Glu Glu Glu Thr Pro His Ser Thr Ser
   1               5                   10
<210> 38
   <211> 12
   <212> PRT
   <213> Homo sapiens
<400> 38
   His Glu Glu Glu Glu Thr Pro His Ser Thr Ser Thr
   1               5                   10
<210> 39
   <211> 35
   <212> PRT
   <213> Homo sapiens
<400> 39
   Pro Gly Gln Leu Ser Phe Glu Val Gln Trp Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 40
   <211> 35
   <212> PRT
   <213> Homo sapiens
<400> 40
   Pro Gly Ala Leu Ser Phe Glu Val Gln Trp Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 41
   <211> 35
   <212> PRT
   <213> Homo sapiens
<400> 41
   Pro Gly Gln Ala Ser Phe Glu Val Gln Trp Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 42
   <211> 35
   <212> PRT
   <213> Homo sapiens
<400> 42
   Pro Gly Gln Leu Ala Phe Glu Val Gln Trp Glu Thr Ser Glu Thr Leu
   1               5                   10                  15
   Lys Ile Leu Val Lys Pro Leu Val Phe Val Cys Arg Glu Val Tyr Asp
                    20                   25                  30
   Pro Pro Cys
           35
<210> 43
   <211> 35
   <212> PRT
   <213> Homo sapiens
<400> 43
   Pro Gly Gln Leu Ser Ala Glu Val Gln Trp Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 44
   <211> 35
   <212> PRT
   <213> Homo sapiens
<400> 44
   Pro Gly Gln Leu Ser Phe Ala Val Gln Trp Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 45
   <211> 35
   <212> PRT
   <213> Homo sapiens
<400> 45
   Pro Gly Gln Leu Ser Phe Glu Ala Gln Trp Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 46
   <211> 35
   <212> PRT
   <213> Homo sapiens
<400> 46
   Pro Gly Gln Leu Ser Phe Glu Val Ala Trp Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 47
   <211> 35
   <212> PRT
   <213> Homo sapiens
<400> 47
   Pro Gly Gln Leu Ser Phe Glu Val Gln Ala Glu Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 48
   <211> 35
   <212> PRT
   <213> Homo sapiens
<400> 48
   Pro Gly Gln Leu Ser Phe Glu Val Gln Trp Ala Thr Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 49
   <211> 35
   <212> PRT
   <213> Homo sapiens
<400> 49
   Pro Gly Gln Leu Ser Phe Glu Val Gln Trp Glu Ala Ser Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 50
   <211> 35
   <212> PRT
   <213> Homo sapiens
<400> 50
   Pro Gly Gln Leu Ser Phe Glu Val Gln Trp Glu Thr Ala Glu Thr Leu
   1               5                   10                  15
   Lys Ile Leu Val Lys Pro Leu Val Phe Val Cys Arg Glu Val Tyr Asp
               20                  25                  30
   Pro Pro Cys
           35
<210> 51
   <211> 54
   <212> PRT
   <213> Homo sapiens
<400> 51
   Pro Gly Gln Pro Leu Ala Ala Val Gln Val Ser Phe Ala Pro Asn Asn
   1               5                   10                  15
   Thr Gly Lys Glu Val Thr Val Glu Cys Lys Ile Asp Gly Ser Ala Asn
               20                  25                  30
   Leu Lys Ser Gln Asp Asp Arg Asp Lys Phe Leu Gly Arg Val Met Phe
           35                  40                  45
   Lys Ile Thr Ala Arg Ala
       50
<210> 52
   <211> 51
   <212> PRT
   <213> Homo sapiens
<400> 52
   Pro Gly Leu Gln Pro Leu Leu Ala Val Gln Phe Thr Asn Leu Thr Met
   1               5                   10                  15
   Asp Thr Glu Ile Arg Ile Glu Cys Lys Ala Tyr Gly Glu Asn Ile Gly
               20                  25                  30
   Tyr Ser Glu Lys Asp Arg Phe Gln Gly Arg Phe Asp Val Lys Ile Glu
           35                  40                  45
   Val Lys Ser
       50
<210> 53
   <211> 29
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(26)
   <223> Xaa can be any naturally occurring amino acid
<400> 53
   Pro Gly Leu Gln Pro Ser Phe Ala Val Gln Val Xaa Xaa Ser Xaa Gln
   1               5                   10                  15
   Xaa Xaa Xaa Xaa Xaa Xaa Xaa Xaa Xaa Xaa Ala Ile Glu
               20                  25
<210> 54
   <211> 30
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(27)
   <223> Xaa can be any naturally occurring amino acid
<400> 54
   Pro Gly Leu Gln Pro Ser Phe Ala Val Gln Val Xaa Xaa Ser Xaa Gln
   1               5                   10                  15
   Xaa Xaa Xaa Xaa Xaa Xaa Xaa Xaa Xaa Xaa Xaa Ala Ile Glu
               20                  25                  30
<210> 55
   <211> 31
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(28)
   <223> Xaa can be any naturally occurring amino acid
<400> 55
   Pro Gly Leu Gln Pro Ser Phe Ala Val Gln Val Xaa Xaa Ser Xaa Gln
   1               5                   10                  15
   Xaa Xaa Xaa Xaa Xaa Xaa Xaa Xaa Xaa Xaa Xaa Xaa Ala Ile Glu
               20                  25                  30
<210> 56
   <211> 32
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(29)
   <223> Xaa can be any naturally occurring amino acid
<400> 56
   Pro Gly Leu Gln Pro Ser Phe Ala Val Gln Val Xaa Xaa Ser Xaa Gln
   1               5                   10                  15
   Xaa Xaa Xaa Xaa Xaa Xaa Xaa Xaa Xaa Xaa Xaa Xaa Xaa Ala Ile Glu
               20                  25                  30
<210> 57
   <211> 33
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(30)
   <223> Xaa can be any naturally occurring amino acid
<400> 57
   Pro Gly Leu Gln Pro Ser Phe Ala Val Gln Val Xaa Xaa Ser Xaa Gln
   1               5                   10                  15
   Xaa Xaa Xaa Xaa Xaa Xaa Xaa Xaa Xaa Xaa Xaa Xaa Xaa Xaa Ala Ile
               20                  25                  30
   Glu
<210> 58
   <211> 29
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(26)
   <223> Xaa can be any naturally occurring amino acid
<400> 58
   Pro Gly Leu Gln Pro Ser Phe Ala Val Gln Val Xaa Xaa Ala Xaa Gln
   1               5                   10                  15
   Xaa Xaa Xaa Xaa Xaa Xaa Xaa Xaa Xaa Xaa Ala Ile Glu
               20                  25
<210> 59
   <211> 30
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(27)
   <223> Xaa can be any naturally occurring amino acid
<400> 59
   Pro Gly Leu Gln Pro Ser Phe Ala Val Gln Val Xaa Xaa Ala Xaa Gln
   1               5                   10                  15
   Xaa Xaa Xaa Xaa Xaa Xaa Xaa Xaa Xaa Xaa Xaa Ala Ile Glu
               20                  25                  30
<210> 60
   <211> 31
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(28)
   <223> Xaa can be any naturally occurring amino acid
<400> 60
   Pro Gly Leu Gln Pro Ser Phe Ala Val Gln Val Xaa Xaa Ala Xaa Gln
   1               5                   10                  15
   Xaa Xaa Xaa Xaa Xaa Xaa Xaa Xaa Xaa Xaa Xaa Xaa Ala Ile Glu
               20                  25                  30
<210> 61
   <211> 32
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(29)
   <223> Xaa can be any naturally occurring amino acid
<400> 61
   Pro Gly Leu Gln Pro Ser Phe Ala Val Gln Val Xaa Xaa Ala Xaa Gln
   1               5                   10                  15
   Xaa Xaa Xaa Xaa Xaa Xaa Xaa Xaa Xaa Xaa Xaa Xaa Xaa Ala Ile Glu
               20                  25                  30
<210> 62
   <211> 33
   <212> PRT
   <213> Homo sapiens
<220>
   <223> Consensus sequence
<220>
   <221> misc_feature
   <222> (12)..(13)
   <223> Xaa can be any naturally occurring amino acid
<220>
   <221> misc_feature
   <222> (15)
   <223> Xaa can be any naturally occurring amino acid
<220>
   <221> misc_feature
   <222> (17)..(30)
   <223> Xaa can be any naturally occurring amino acid
<400> 62
   Pro Gly Leu Gln Pro Ser Phe Ala Val Gln Val Xaa Xaa Ala Xaa Gln
   1               5                   10                  15
   Xaa Xaa Xaa Xaa Xaa Xaa Xaa Xaa Xaa Xaa Xaa Xaa Xaa Xaa Ala Ile
               20                  25                  30
   Glu

## Description

### FIELD OF THE INVENTION

The present invention relates to a protein capable of binding to the domain encoded by exon 9 of human CD44 (CD44ex9), and to fusion proteins and conjugates of said protein. The invention further relates to a method of production for the protein and the protein or conjugate of the invention for the use in treatment and diagnosis of various diseases, and especially for cancer.

### BACKGROUND OF THE INVENTION

Extensive studies of cancer transcriptional patterns have led to the discovery of molecular targets to distinguish the malignant from the benign and the most aggressive cancers from those that are less aggressive. Cancers often overexpress a number of proteins, including certain cell surface antigens, e.g. cell surface receptors. Antibodies that bind such overexpressed cell surface antigens can facilitate detection and treatment of such cancers. A number of approaches have been utilized to generate antibodies to cancer cell surface receptors which can be used as potential diagnostics or therapeutics. Identification of overexpressed cell surface receptors and antibodies which bind them provide a route to the development of cancer therapies, especially for those cancer subtypes with poor prognosis and resistance to traditional therapies. CD44, CA19-9 and CEA are such overexpressed cell surface receptors, which are known to be relevant for tumor progression and malignancy.

CD44, a major adhesion molecule for the extracellular matrix, has been implicated in a wide variety of physiological processes, including leukocyte homing and activation, wound healing, and cell migration, as well as in tumor cell invasion and metastasis (Günthert et al., 1991; Nagano and Saya, 2004; Ponta et al., 2003).

CD44 is a single chain molecule comprising a conserved N-terminal extracellular domain, a non-conserved membrane proximal region, a variable region expressing various combinations of variant exons, a conserved transmembrane spanning domain and a conserved cytoplasmic tail. The genomic map of CD44 includes 5 constant exons at the 5' terminus and 5 constant exons in the 3' end. The mouse CD44 gene includes also 10 variant exons in the middle of the molecule designated V1-V10 resulting in a total of 20 exons. The human CD44 gene comprises only 9 of these 10 variant exons (V2-V10) thus comprising a total of 19 exons. Differential alternative splicing generates many isoforms of CD44 that express various combinations of variant exons (designated Exon Vₓ, x = 1 to 10), which are inserted in the membrane proximal domain and constitute the variable region of the molecule. These molecules are designated CD44 variants (CD44v). To date, 20 isoforms of CD44 are known.

Whereas the standard CD44 isoform (CD44s) is expressed predominantly in hematopoietic cells and normal epithelial cell subsets, the CD 44 variants with insertions in the membrane-proximal extracellular region were found to be abundant in a variety of human tumors, including colon, mammary, gastric, bladder, prostate carcinomas, and various hematopoietic neoplasms. Additional reports suggested a close correlation between expression of the variant CD44 and tumor progression and in particular the lymphatic spread of neoplastic cells.

Taken together, these observations point to an important function of CD44 variants in tumor initiation and the maintenance of cancer cells in addition to its more established functions in cell adhesion and migration.

Beside the role of CD44 in cancer, CD44 has also been suggested as a potential target in autoimmune diseases. It has been reported (Hale et al., 1992) that administration of a CD44 protein or peptide or derivative can be used for treating various autoimmune diseases.

Based on the established role of CD44 in cancer and autoimmune diseases, monoclonal antibodies directed against various variant regions of CD44 have also been generated as potential agents for diagnosis or therapy of CD44-related disorders.

Seiter et al. describe mAbs directed against metastasis-specific variants of CD44 surface protein of a rat pancreatic adenocarcinoma (Seiter et al., 1993). These antibodies are proposed for producing immunosuppression for therapeutic treatment of immunoregulatory disorders including, for example, diseases of the rheumatic type. Monoclonal antibodies reactive with CD44 which inhibit T-cell proliferation were also provided for treatment of various autoimmune diseases. Monoclonal antibodies binding to forms of CD44 containing the exon v6 peptide were also reported as being useful for diagnosing inflammatory diseases (Jalkanen et al., 1986).

The WO 91/17248 A1 relates to the use of anti-CD44v antibodies for the therapy and diagnosis of tumors. WO 95/00851 A1 relates to the use of antibodies directed against the variable exons of CD44 for diagnosis of tumors. The WO 95/04547 A1 discloses the use of anti-CD44 antibodies especially directed against epitopes within the sequence of exon v5 for immunotherapeutic and immunoscintigraphic purposes. An anti-CD44 antibody directed against exon v6 is disclosed by WO 95/33771 A1. Furthermore, the EP 0 538 754 A2 teaches the use of antibodies directed against CD44 variants for immunosuppression.

The anti-CD44 antibodies of the prior art show several disadvantages. They represent large and complex molecules which have to be generated by recombinant production methods. Hence, the production process is elaborate, costly and has to fulfill strict regulatory requirements. Furthermore, the necessity for a reduced immunogenic potential requires the generation of human or humanized antibodies.

The database entry no. ADR58907, retrieved from EBI accession no. GSP:ADR58907 (DATABASE Geneseq [Online] 18 November 2004, "Human Elk1 phosphorylation/Eik1 kinase activation protein- SEQ ID 10.") and WO 2004/072277 A2 (ASAHI KASEI PHARMA CORP [JP]; MATSUZAKI OSAMU [JP]; MATSUDA AKIO [JP]; 26 August 2004) disclose a peptide of 184 amino acids which is 100% identical to SEQ ID No. 2 of the present application in a 32 aa overlap. Notably, this database entry does not teach any binding to CD44.

The database entry. No. AWV72632 (retrieved from EBI accession no. GSP:AWV72632, DATABASE Geneseq [Online] 9 July 2009, "Human pancreatic cancer associated target (PCAT) protein, SEQ ID14.") and US 2009/138977 A1 (DOMON BRUNO [US] ET AL; 28 May 2009) disclose a protein of 198 amino acid which comprises aa 33 to 66 of SEQ ID. No. 4 of the present invention with only 1 amino acid difference. For said protein no binding to CD44 is disclosed.

The database entry No. AZM18611 (retrieved from EBI accession no. GSP: AZM18611, DATABASE Geneseq [Online] 13 October 2011, "Human lung cancer target LCAT protein sequence SEQ: 1.") and US 2011/212085 A1 (JOSELOFF ELIZABETH [US] ET AL; 1 September 2011) disclose a human lung cancer target LCAT protein of 102 amino acids comprising complete 62 aa long SEQ ID NO: 5 of the application. There is no disclosure of binding to CD44.

The application WO 1995/04547 A1 (Boehringer Ingelheim Int. [OE]; Kernforschungszentrum Karlsruhe [OE]; Adolf G) 16 February 1995) discloses proteins and peptides binding to the V5 domain of CD44, which are, however, completely different to the amino acid sequences of the present invention.

Kenichiro et al. ("Inhibition of liver metastasis formation by anti CD44 variant exon 9 monoclonal antibody", Int. J. Oncology, 1997, vol. 11, no. 6, pages 1257-1261) discloses the ability of a monoclonal antibody directed against CD44 variant exon 9 to inhibit liver metastasis formation. However, Kenichiro et al. is silent on peptides binding to CD44 exon 9.

Kimura et al, 2013 ("CD44variant exon 9 plays an important role in colon cancer initiating cells", Oncotarget, pages 785-791) pronounces the important role of the CD44variant exon 9 for the initiation of colon cancer but does not disclose CD4ex9 binding peptides.

Park et al. ("Screening of Peptides Bound to Breast Cancer Stem Cell Specific Surface Marker CD44 by Phage Display", Molecular Biotechnology, 2011, vol. 51, no. 3, pages 212-220) relates of a phage display peptide screening against CD44 but does not disclose any peptide related to the peptides of the present invention.

Database entry No. AZV55426 (retrieved from EBI accession no. GSP: AZV55426; database Geneseq [Online] 21 June 2012, "Human derived cancer-associated target protein SEQ ID NO: 673.") and US 8 168 586 B1 (Fang Dong et al., 1 May 2012) disclose a sequence with 57 aa and having 83.3% identity to the whole sequence of SEQ ID No: 4 of the present invention. Said protein is identified as a human cancer-associated target protein. However, there is no reference of binding to CD44 exon 9.

Database entry No. C9J6S2 (UniProt Online database, 3 November 2009; "SubName: Full = Sodium/potassium-transporting ATPase subunit beta-3 {EC 0:00003131 Ensembl: ENSP00000418353};" retrieved from EBI accession no. UNIPROT:C9J6S2) and Burkhard et al. ("Initial characterization of the human central proteome", BMC Systems Biology 2011, vol. 5, no. 1, page 17) disclose a human Sodium/potassium-transporting ATPase subunit beta-3 with 89 aa and having 83.3% identity to the whole sequence of SEQ ID No: 4 of the present invention. There is no reference of binding to CD44 exon 9.

Database entry No. AXT76926 (retrieved from EBI accession no. GSP: AXT76926; Geneseq Online, 18 February 2010; "Human Na-K ATPase beta3 CAT protein SEQ ID:130") and US 7 582 441 B1 (Ruben S et al., 1 September 2009) disclose a human Na-K ATPase beta3 CAT protein of 57 aa and having 89.5 % identity to the whole sequence of SEQ ID No: 4 of the present invention. There is no reference of binding to CD44 exon 9.

Database entry No. ARW93074 (retrieved from EBI accession No. GSP: ARW9307; Geneseq Online 7 August 2008, "N-linked glycopeptide SEQ: 101."), database entry No. ARW9307 (retrieved from EBI accession No. GSP: ARW93073, database Geneseq Online, 7 August 2008, "N-linked glycopeptide SEQ: 100.") and WO 2008/066629 A2 (Inst Systems Biology, Aebersold R, Zhang H, 5 June 2008) disclose sequence ID No: 100 of 25 amino acids and sequence ID No: 101 of 32 amino acids both having 54.9% identity to the whole sequence of SEQ ID No: 4.

Database entry No. AAM62836 (retrieved from EBI accession No. GSP: AAM62836, database Geneseq Online, 5 November 2001, "Human brain expressed single exon probe encoded protein SEQ ID NO: 34941.") and WO 01/57275 A2 (Molecular Dynamics Inc.; Penn Sharron G; Hanzel Oavio K; 9 August 2001) disclose a protein of 63 amino acids, expressed in human brain and having an identity of 61.3% to the SEQ ID No: 5 of the present invention.

Hence, there is still the need for CD44 variant-binding molecules. The objective of the present invention thus is to provide a CD44 variant binding substance which overcomes at least one of the above mentioned disadvantages.

This problem is solved by provision of a CD44-variant binding protein according to claim 1 and a use of said binding protein for diagnosis and therapy. Specific embodiments of the invention are subject matter of further independent or dependent claims.

### SUMMARY OF THE INVENTION

In a first aspect the invention provides a protein capable of binding to a polypeptide encoded by exon 9 of human CD44 (CD44ex9), said protein comprises or consists of
a) an amino acid sequence according SEQ ID No. 1 to 5; and SEQ ID No. 39 to 52; or
b) an amino acid sequence with at least 85% identity to the amino acid sequence given in SEQ ID No. 1 to 5, SEQ ID No. 39 to 50, and SEQ ID No. 52 as a whole; or
c) an amino acid sequence with at least 95% identity to the amino acid sequence given in SEQ ID No. 51 as a whole; and
wherein said protein has a length of 100 amino acids or less.

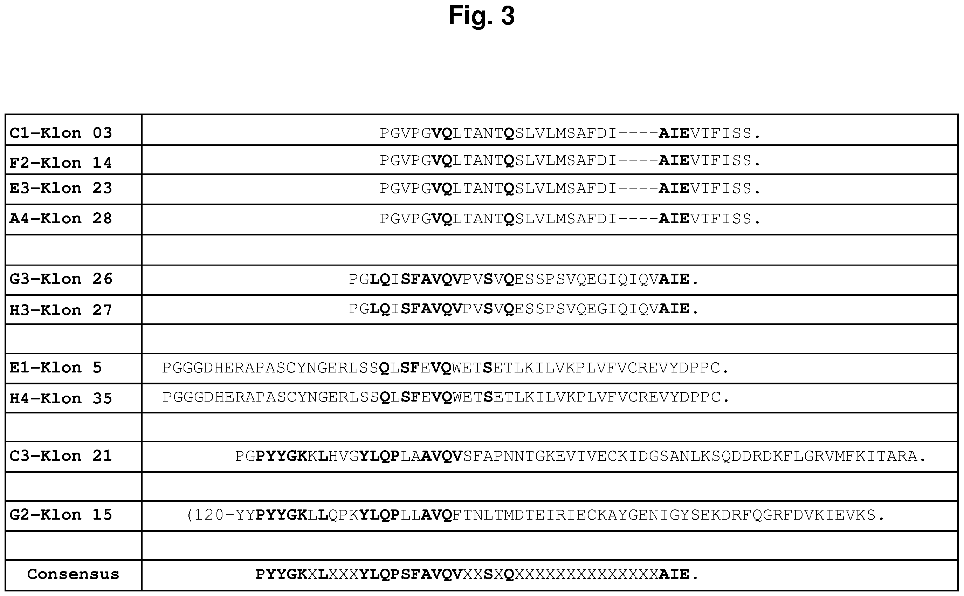

By performing a yeast-two hybrid-screening the inventors were able to identify five different peptides with a specific binding to the polypeptide encoded by exon 9 of CD44. The amino acid sequences are presented in Figure 3 and in the following table and designated as SEQ ID No. 1 to 5.

| **SEQ ID No.** | **Amino acid sequence** |
|---|---|
| 1 | PGVPGVQLTA NTQSLVLMSA FDIAIEVTFI SS |
| 2 | PGLQISFAVQ VPVSVQESSP SVQEGIQIQV AIE |
| 3 | |
| 4 | |
| 5 | |

A further analysis revealed that a consensus sequence can be derived from these sequences exhibiting the following amino acid sequence:
PYYGKXLX₃YLQPSFAVQVX₂SXQX₁₀₋₁₄AIE.

These consensus sequences are designated as SEQ ID No. 6 to 10 as depicted in the following table whereby these five sequences differ in the length of the arbitrary sequence X₁₀₋₁₄. Please note that "X" is defined as an arbitrary amino acid.

| **SEQ ID No.** | **Amino acid sequence** |
|---|---|
| 6 | PYYGKXLXXX YLQPSFAVQV XXSXQXXXXX XXXXXAIE |
| 7 | PYYGKXLXXX YLQPSFAVQV XXSXQXXXXX XXXXXXAIE |
| 8 | PYYGKXLXXX YLQPSFAVQV XXSXQXXXXX XXXXXXXAIE |
| 9 | PYYGKXLXXX YLQPSFAVQV XXSXQXXXXX XXXXXXXXAI E |
| 10 | PYYGKXLXXX YLQPSFAVQV XXSXQXXXXX XXXXXXXXXA IE |

The ability to derive a unique consensus sequence which encompasses all independent isolated CD44-interacting peptides further validates the results of the two-hybrid screening method.

In subsequent two independent affinity assays the peptides of SEQ ID No. 1 to 5 were analysed for binding towards the polypeptide encoded by exon 9 of CD44 and allowed an affinity ranking of the peptides, whereby the peptides were named as follows:
Peptide of SEQ ID No. 1: Peptide A
Peptide of SEQ ID No. 2: Peptide C
Peptide of SEQ ID No. 3: Peptide B
Peptide of SEQ ID No. 4: Peptide D
Peptide of SEQ ID No. 5: Peptide E

Both affinity assays, the pseudohitpicking assay and the Fluorescein di-beta-D-galactopyranoside (FDG)-based assay revealed the same ranking order:
B> A,C,D,E
C> A,D,E
D> A,E
E> A
A>

In sum, peptide B (= SEQ ID No. 3) possesses the highest affinity towards the polypeptide encoded by exon 9 of CD44 and has to be regarded as preferred peptide in the context of the invention. For this peptide an epitope mapping was performed, whereby a deletion analysis and an alanine scan was performed.

Furthermore, also the peptides A, C, D and E were analysed in a deletion assay, confirming the validity of the consensus sequence.

Based on these analyses, the invention provides in a further aspect a protein binding to a polypeptide encoded by exon 9 of human CD44 (CD44ex9), said protein comprises or consists of
a) an amino acid sequence according SEQ ID No. 39 to 52; or
b) an amino acid sequence with at least 85%, more preferably 90% and most preferably 95% identity to the amino acid sequence given in SEQ ID No. 39 to 52; and
wherein said protein has a length of 100 amino acids or less.

| **SEQ ID No.** | **Amino acid sequence** |
|---|---|
| 39 | PGQLSFEVQW ETSETLKILV KPLVFVCREV YDPPC |
| 40 | PGALSFEVQW ETSETLKILV KPLVFVCREV YDPPC |
| 41 | PGQASFEVQW ETSETLKILV KPLVFVCREV YDPPC |
| 42 | PGQLAFEVQW ETSETLKILV KPLVFVCREV YDPPC |
| 43 | PGQLSAEVQW ETSETLKILV KPLVFVCREV YDPPC |
| 44 | PGQLSFAVQW ETSETLKILV KPLVFVCREV YDPPC |
| 45 | PGQLSFEAQW ETSETLKILV KPLVFVCREV YDPPC |
| 46 | PGQLSFEVAW ETSETLKILV KPLVFVCREV YDPPC |
| 47 | PGQLSFEVQA ETSETLKILV KPLVFVCREV YDPPC |
| 48 | PGQLSFEVQW ATSETLKILV KPLVFVCREV YDPPC |
| 49 | PGQLSFEVQW EASETLKILV KPLVFVCREV YDPPC |
| 50 | PGQLSFEVQW ETAETLKILV KPLVFVCREV YDPPC |
| 51 | |
| 52 | |

Based on this epitope mapping of the peptides the above disclosed consensus sequence in general was confirmed. However, a more preferred consensus sequence can be derived from these sequences exhibiting the following amino acid sequence:
PGLQPSFAVQVX₂^{S}/_{A}XQX₁₀₋₁₄AIE.

These consensus sequences are designated as SEQ ID No. 53 to 62 as depicted in the following table whereby these sequences differ in an amino acid exchange at position 14 (Ser-Ala) and the length of the arbitrary sequence X₁₀₋₁₄. Please note that "X" is defined as an arbitrary amino acid.

| **SEQ ID No.** | **Amino acid sequence** |
|---|---|
| 53 | PGLQPSFAVQ VXXSXQXXXX XXXXXXAIE |
| 54 | PGLQPSFAVQ VXXSXQXXXX XXXXXXXAIE |
| 55 | PGLQPSFAVQ VXXSXQXXXX XXXXXXXXAI E |
| 56 | PGLQPSFAVQ VXXSXQXXXX XXXXXXXXXA IE |
| 57 | PGLQPSFAVQ VXXSXQXXXX XXXXXXXXXX AIE |
| 58 | PGLQPSFAVQ VXXAXQXXXX XXXXXXAIE |
| 59 | PGLQPSFAVQ VXXAXQXXXX XXXXXXXAIE |
| 60 | PGLQPSFAVQ VXXAXQXXXX XXXXXXXXAI E |
| 61 | PGLQPSFAVQ VXXAXQXXXX XXXXXXXXXA IE |
| 62 | PGLQPSFAVQ VXXAXQXXXX XXXXXXXXXX AIE |

It has to be emphasized that the peptides of the invention show for the first time that not only large antibodies can be generated against CD44 epitopes but also rather small peptides with a length between 32 and 66 amino acids which bind specifically and selectively to a clinically relevant CD44 domain.

In contrast to the (monoclonal) CD44v5 antibodies of the prior art, these peptides exhibit several advantages.

Since they represent short peptides without the necessity to form complexes or intramolecular disulfide bridges they are much easier to produce and to purify.

As peptides with a length of between 32 and 66 amino acids it is even possible to synthesize them by solid phase synthesis (according Merrifield). This *ex vivo*-protein synthesis is especially of advantage given the strict regulatory requirements associated with recombinant protein expression.

Furthermore, as small peptides they can be advantageously conjugated or fused to a different protein, specific compounds or even nanoparticles in order to be used for therapy or diagnosis.

As small peptides they are able to cross the blood brain barrier and cellular membranes to deliver compounds to compartments which could not be addressed by antibodies.

Due to their small size the proteins of the inventions the risk to elicit an immune response is strongly reduced. It has to be remarked that the risk of immunogenicity which is inherent for antibodies markedly impairs their clinical use.

It has to be emphasized that the CD44ex9-binding protein of the invention by interfering only with the variant domain v5 leaves the normal CD44 function untouched and therefore allows a specific intervention.

In sum the CD44ex9-binding proteins of the invention open the door to novel strategies for therapy and diagnosis of cancer with a reduced risk of side effects.

### DETAILED DESCRIPTION OF THE INVENTION

The invention is set out in the appended set of claims.

In one embodiment of the invention the invention provides a protein capable of binding to a polypeptide encoded by exon 9 of human CD44 (CD44ex9), said protein comprises or consists of an amino acid sequence with at least 85% identity, preferably at least 85%, more preferably least 90%, most preferably at least 95% identity and specifically at least 97.5% identity to the amino acid sequence given in SEQ ID No. 1 to 5 and 39 to 52 as a whole.

In a further aspect the application discloses a protein binding to a polypeptide encoded by exon 9 of human CD44 (CD44ex9), said protein comprises or consists of an amino acid sequence with at least 90% identity, preferably at least 95%, more preferably at least 97.5% and even more preferably at least 100% identity to the CD44-binding motif defined by the sequence: "PYYGKXLX₃YLQPSFAVQVX₂SXQX₁₀₋₁₄AIE" as depicted in SEQ ID Nos. 6 to 10, and "PGLQPSFAVQVX₂^{S}/_{A}XQX₁₀₋₁₄AIE" as depicted in SEQ ID Nos. 53 to 62, wherein X denotes an arbitrary amino acid.

The proteins as claimed herein have a length of 100 amino acids or less and even more preferably a length of 90, 85, 80, 75, 70 or 66 amino acids or less.

In a further embodiment of the invention the CD44ex9-binding protein has a high affinity to the polypeptide encoded by exon9 with a kᵢ value of less than 10 µM, preferably of less than 1 µM, more preferably of less than 100nM and most preferably of less than 10 nM.

In a further embodiment of the invention the CD44ex9-binding protein binds selectively to the polypeptide encoded by exon9, which is given when it binds to other arbitrary proteins with a kᵢ value which is preferably at least 10 times less and more preferably 100 times less of the ki value for CD44ex9-binding.

In one embodiment the CD44ex9-binding protein of the invention is capable to bind to every CD44 isoform that contains the domain encoded by exon 9, which is also designated as "variant 5" (v5).

In a preferred embodiment the CD44ex9-binding protein of the invention binds to a CD44 isoform selected from the list consisting of CD44 v5, CD44 v5 -v6, CD44 v3 - v6, CD44 v2 - v10, CD44 v3 - v10, CD44 V4 - v7 and CD44 v4 - v10.

Notably, the CD44ex9-binding protein of the invention binds also to proteins that comprise beside the domain v5 also other variant domains such as v1, v2, v3, v4, v6, v7, v8, v9 and/or v10.

In a further embodiment of the invention the CD44ex9-binding protein of the invention is conjugated or fused to a heterologous protein or polypeptide.

The binding protein can be preferably linked or fused to a protein selected from the group consisting of antibodies, toxins and cytokines.

In a preferred embodiment the conjugated/fused protein is an enzyme which catalyses the generation of a cytotoxic or cytostatic agent from a precursor molecule. A non-exhaustive lists of suitable enzymes contains the aldehyde-oxidase, amino acid oxidase, cytochrome P450 oxidase, NAD(P)H:quinone oxidoreductase, tyrosinase, thymidilate synthase, thymidine phosphorylase, glutathione-S transferase, deoxycytidine kinase, carboxylesterase, alkaline phosphatase, beta-glucuronidase, cysteine conjugate-beta lyase, and nitroreductase.

The binding protein of the invention might also be conjugated or fused to a protein that inhibits, impairs or kills the cancer cell or makes it sensitive to a further cytotoxic compound selected from the list consisting of cytosine deaminase, soluble Fms-like tyrosine kinase ligand, herpes simplex virus-1 thymidine kinase (HSV1-TK), cytochrome P450 2B1, retinoblastoma related proteins, p16/cdkn2 and MMAC1/PTEN. MMAC1/PTEN acts as a negative regulator of the phosphor-inositide 3-kinase. MMAC1 stands for Mutated in Multiple Advanced Cancers 1.

In a further aspect the invention provides a conjugate comprising the CD44ex9-binding protein of the invention which is linked directly or indirectly by a linker to a compound selected from the group consisting of a carbohydrate, a dye molecule, a radioactive isotope, a toxin, a cytostatic agent, a cytokine, a immunomodulatory agent, or a prodrug thereof.

The conjugate according to invention can in general be linked to any type of drug which impairs, inhibits or even kills cancer cells. Hence, a cytotoxic agent is particularly preferred. Non-limiting examples for cytotoxic agents include emozolomide, carmustine, lomustine, procarbazine, streptozocin, irinotecan or any combination of two or more of these agents.

Further examples for suitable cancer inhibitors are (-)-Ci-Cdp1, (-)-Ci-Cdp2, (-)-epigallocatechin gallate, (+)-Cbi-Cdpi2, (+)-Ci-Cdp2, 10-Deacetylbaccatin III, 4-demethoxy daunorubicin, 5-azacytidine/5-aza-2'-deoxycytidine, 5-fluorouracil, 5-iminodoxorubicin hydrochloride, 6-mercaptopurine, aclarubicin, acodazole, actinomycin D, adenine phosphate, adenosine, aderbasib, adozelesin; U-73, 975, afeletecan, alemtuzumab, alitreninoin, alosetron HCI, alphitolic acid, altretamine, alvespimycin, ambazone, ametantrone, amifostine, aminoglutethimide, amsacrine HCI, amsilarotene, amygdalin, anagrelide, anastrozole, anaxirone, ancitabine, annomontacin, annomuricin A, (C19/C20-Erythro), annomuricin B, (C10/C11, C19/C20-Erythro), annomuricin C, (All Threo) annomuricin E, annonacin, annonacin-IO-One, annonacin-A-One, annonidin B, annonin VI, annosquamosin A, annosquamosin B, antramycin, apaziquone, argimesna, aristoforin, arsenic trioxide, artemisinin, ascomycin, asparaginase, atosiban, atrimustine, axitinib, azasetron HCI, azatepa, azathioprine, azotomycin, bafetinib, balamapimod, banoxantrone, batabulin, batimastat, Bbr-34384, becatecarin, belotecan, benaxibine, bendamustine, benzodepa, berubicin, betulin, betulinic acid, betulinic aldehyde, bevacizumab, bexarotene, bicalutamide, bietaserpine, biricodar, bisantrene, bistramid A; bistratene A, bizelesin, bleomycin, bleomycin A2 [Sulfate], bleomycin A5, bleomycin Sulfate, bortezomib, bosentan, bosutinib, brequinar sodium, brequinar, bropirimine, brostallicin, budotitane, bullatacin, buserelin, busulfan, cabazitaxel, calcium folinate, calcium levofolinate, calusterone, camptothecin, canertinib, canfosfamide, cantharidin, capecitabine, caracemide, carbetimer, carboplatin, carboprost (carboprost tromethamine), carboquone, carfilzomib, carglumic acid, carmofur, carzelesin, cedefingol, cemadotin, cetuximab, cevipabulin, chlorambucil, chlormethine (mechlorethamine), chlorotamoxifen, chlorotrianisene, cioteronel, cisplatin, cladribine, clanfenur, clofarabine, clofazimine, clomifene citrate, cordycepin, corosolic acid, crisnatol, curcumin, cyclocytidine, cyclophosphamide, cytarabine, cytidine, D-aminolevulinic acid, dacarbazine, damsin, daniquidone, danusertib, daporinad, darinaparsin, dasatinib, daunoblastin, daunorubicin/ daunomycin, decitabine, deferasirox, deforolimus, demecolcine, denibulin, detorubicin, dexniguldipine, dexormaplatin, dezaguanine, dianhydrodulcitolum, dibrospidium chloride, dienogest, diflomotecan, dinalin, disermolide, docetaxel, dofequidar, dolasetron mesylate, dovitinib, doxifluridine, doxorubicin, dromostanolone, duazomycin, duocarmycin, dynemicin, ecomustine, edatrexate, edotecarin, edotreotide, eflornithine, elacridar, eacytarabine, elesclomol, elinafide, elomotecan, elsamitrucin, emitefur, enloplatin, enocitabine, enpromate, entecavir, entinostat, entricitabine, enzastaurin, epirubicin, eptaloprost, eribulin, erlotinib, Esorubicin, estramustine, etalocib, etanidazole, etoglucid, etoposide, exatecan, exemestane, exisulind, fadrozole, fazarabine, fiacitabine, floxuridine, fludarabine, fluoxymesterone, fluorocitabine, flutamide, formestane, forodesine, fosfluridine tidoxil, fosquidone, fostriecin, fotemustine, fotretamine, fulvestrant, fumagillin, galarubicin, galocitabine, gefitinib, gemcitabine, gemtuzumab ozogamicin, geroquinol, gigantetronenin, gigantetroneninone, gimatecan, gimeracil, gloxazone, glufosfamide, goniothalamicin, goniothalamicinone, goserelin, granisetron HCI, gusperimus, hexarelin, homoharrlngonine, hydrocamptothecine, hydroxy carbamide, hydroxyurea, hypericin, ibandronate sodium, ibandronic acid, idarubicin HCI, idronoxil, ifosfamide, ilmofosine, imatinib, imatinib mesylate, imexon, improsulfan, incadronate, indibulin, indisulam, inolitazone, inproquone, intiquinatine, intoplicine, iobenguane, irofulven, irsogladine, ispinesib, ixabepilone, ketotrexate, L-alanosine, laniquidar, lapatinib ditosylate, laromustine, larotaxel, ledoxantrone, lenalidomide, lentinan, lestaurtinib, letrozole, leuprolide acetate, leuprorelin, lexacalcitol, liarozole, lobaplatin, lonafarnib, lonidamine, losoxantrone, Ly-83583, lysipressin, mafosfamide, mannomustine, mannosulfan, marimastat, marinomycin A, masitinib, maslinic acid, masoprocol, mechlorethamine, medorubicin, megestrol, mepitiostane, mercaptopurine, mesna, methotrexate, methyl aminolevulinate, metomidate, metoprine, meturedepa, miboplatin, midostaurin, mifamurtide, milataxel, miproxifene, miriplatin, misonidazole, mitindomide, mitoflaxone, mitoguazone, mitomycin, mitonafide, mitoquidone, mitotane, mitoxantrone, mitozolomide, mivobulin, mizoribine, mofarotene, mopidamol, motesanib, motexafin, mubritinib, muricapentocin, muricatacin, mustine HCI, mycophenolate mofetil, mycophenolic acid, nedaplatin, nelzarabine, nemorubicin, neocuproine, neptamustine, neratinib, nigericin, nilotinib, nilutamide, nimustine, ninopterin, nitracrine, nogalamycin, nolatrexed, norcantharidine, nor-dihydroguaiaretic acid, nortopixantrone, novembichin, obatoclax, octreotide, olaparib, oleanolic aldehyde, omacetaxine mepesuccinate, ombrabulin, omtripolide, ondansetron HCI, ortataxel, oteracil, oteracil potassium, oxaliplatin, oxisuran, oxophenarsine, paclitaxel ceribate, palifosfamide, palonosetron, pamidronate disodium, pamidronic acid, panitumumab, panobinostat, patubilone, pazelliptine, pazopanib, pegaspargase, peldesine, pelitinib, pelitrexol, pemetrexed disodium, pentostatin, peplomycin, peretinoin, perfosfamide, perifosine, pibrozelesin hydrobromide, picoplatin, pinafide, piposulfan, pirarubicin, pirfenidone, piritrexim, piroxantrone, pixantrone, plevitrexed, plicamycin, plitidepsin, plomestane, podophyllotoxin, pomalidomide, porfimer sodium, pralatrexate, prinomastat, procarbazine HC1, propamidine, prospidium chloride, pumitepa, puromycin, pyrazofurin, ouarfloxin, raltegravir, raltitrexed, ramosetron HC1, ranimustine, retaspimycin, retelliptine, riboprine, ritrosulfan, rituximab, roflumilast, romidepsin, ropidoxuridine, roquinimex, rosabulin, rubitecan, sabarubicin, safingol, salirasib, sapacitabine, saracatinib, sardomozide, satraplatin, sebriplatin, seliciclib, semaxanib; SU-5416, semustine, sermorelin, simotaxel, simtrazene, sitagliptin, sizofuran, soblitodin, sobuzoxane, sodium phenylbutyrate, sorafenib, sparfosic acid, sparsomycin, spiroplatin, squalamine, squamocin, streptonigrin, streptovarycin, sufosfamide, sulofenur, sunitinib, swainsonine, tacedinaline, tafluposide, talabostat, talisomycin, tallimustine, talotrexin, taltobulin, tamoxifen citrate, tandutinib, tanespimycin, tariquidar, tasidotin, tasisulam, tauromustine, tegafur, tegafur-uracil, telantinib, teloxantrone, temozolomide, teniposide, tenuazonic acid, terameprocol, teriparatide, tesetaxel, testolactone, tezacitabine, thiamiprine, thioguanine, thiotepa, thymopoietin, tiazofurine, tilomisole, tilorone, timcodar, timonacic, tioguanine, tirapazamine, tocladesine, tomudex, topotecan hydrochloride, toremifene citrate, tosedostat, tositumomab, toxipantrone, trastuzumab, trenimon, tretinoin, triciribine, trilostane, trimetrexate, triplatin tetranitrate, triptolide, triptorelin, trofosfamide, tropisetron HC1, tubulozole, tylophorin, U-67786, U-68415, U-71184, U-76074, U-78057, ubenimex, uramustine, uredepa, urethane, uridine, ursolic acid, ursolic aldehyde, vadimezan, valrubicin, valspodar, vandetanib, vapreotide, vatalanib; PTK-787, verteporfin, vildagliptin, vinblastine sulfate, vincristine, vindesine, vinepidine, vinflunine, vinformide, vinfosiltine, vinleucinol, vinleurosine, vinorelbine [Base], vinorelbine tartrate, vintriptol, vinzolidine, voriconazole, vorinostat, vorozole, wilforlide A, xanthomycin A, zalcitabine, zeniplatin, zilascorb, zinostatin, zoledronic acid, zorubicin, zosuquidar, or the like, or a combination comprising at least one of the foregoing cancer inhibitors.

Furthermore, also an anti-angiogenic drug could be used for the conjugate of the invention. Non-limiting examples are bevacizumab, aflibercept, cediranib, sorafenib, sunitinib, vandetanib, pazopanib, vatalanib, imatinib mesylate, cilengitide, angiostatin, endostatin, platelet factor-4. Particularly, said anti-angiogenic drug could be used in any combination of two or more of the listed examples.

The radiation sensitizing agents represents a further drug class for the conjugate comprising the CD44ex9-binding protein according the invention. Non-limiting examples are carboplatin, cilengitide, CG841251, staurosporine derivatives such as MK-1775, 4-phenylbutyrate, a gadolinium containing compound such as motexafin-gadolinium, a taxane derivative such as paclitaxel or docetaxel or a combination thereof.

In another aspect the invention provides an isolated nucleic acid molecule encoding the CD44ex9-binding protein according to the invention.

In a further aspect the invention is related to an expression vector containing the nucleotide sequence of the CD44ex9-binding protein or a fusion protein thereof.

In a still further aspect the invention provides a host cell containing the expression vector containing the nucleotide sequence of the CD44 ex9 protein or a fusion protein thereof.

In a further embodiment the invention provides a method of producing the CD44ex9-binding protein, wherein said method comprises the following steps:
1.) Transforming a host cell with an expression construct comprising a nucleic acid molecule encoding the protein of the invention or a respective fusion protein; and
2.) Culturing the host cell under conditions suitable for producing the CD44ex9-binding protein.

In one embodiment of the invention the CD44ex9-binding protein can be used for diagnosis or treatment of a disease selected from the group consisting of autoimmune diseases such as insulin-dependent diabetes, multiple sclerosis, Sjögren's syndrome or systemic lupus erythematosus (SLE); skin diseases such as psoriasis or atopic dermatitis; chronic inflammatory diseases such as rheumatoid arthritis or inflammatory bowel disease; tissue injury; allergic diseases and cancer disease.

The use of the CD44ex9-binding protein of the invention for the diagnosis or treatment of inflammation is based on the fact that CD44 plays a role in directing inflammatory cells to the site of infection or tissue destruction. Hereby the CD44 on activated T-lymphocytes, monocytes or activated endothelial cells binds the extracellular matrix component hyaluronan, induces chemokines such as CCR2 which then stimulates the migration of inflammatory cells to the inflammatory site (Johnson & Ruffell, 2009). Blockage of CD44 by the CD44ex9-binding protein would prevent this process.

For example CD44 expression has been extensively studied in patients with rheumatoid arthritis whereby the level of CD44 expression on monocytic cells in the synovial fluid from patients with RA has been shown to be elevated. This increase in CD44 expression was positively correlated with the degree of synovial inflammation in RA. Furthermore, CD44 deficient mice have lower grades of arthritis and anti-CD44 treatment was shown to effectively reduce the arthritis score in animal models. Hence, the CD44-ex9-binding protein of the invention can be used for diagnosis or therapy of RA.

In a further embodiment of the invention the CD44ex9-binding protein can be used for producing immunosuppression, e.g. for prevention of transplant rejection by inhibiting T-cell proliferation.

In a preferred embodiment of the invention the CD44ex9-binding protein can be used for diagnosis or treatment of cancer disease. This preferred use is based on the fact that CD44 variants comprising the v5 domain have been described in cancer cells.

Due to the specific binding of the CD44ex9-binding protein towards the v5 domain of the CD44 protein, said binding protein inhibits the interaction of CD44 with various extracellular matrix proteins and thus inhibits the attachment, migration and metastasis of tumor cells and may also impair the proliferation rate of tumor cells.

In a further preferred embodiment the CD44ex9-binding protein of the invention can be used for the diagnosis or treatment of cancer stem cells (CSC). An emerging concept indicates that CSC ultimately determine the success of cancer treatment since the cells represent a long term surviving population which later on leads to relapses and metastasis (Deonarain et al., 2009). CD44 was shown to be expressed on CSCs and was made responsible for said long term survival and metastatic potential of the respective tumors. As an example the CSC of breast cancer are characterized by the expression of CD44⁺/CD24^{low}, which argues for CD44 as a promising anti CLC-target.

In another embodiment of the invention the CD44ex9-binding protein of the invention can be used for the diagnosis or treatment of chronic lymphatic leukemia (CLL). Zhang et al (2013) could show that CLL cells show a high expression of CD44 and furthermore that anti CD44 antibodies were effective in killing CLL-cells in vitro and in vivo, whereby in a xenograft model the tumor completely disappeared from the organism.

The diagnosis or treatment of breast cancer by the CD44ex9 binding protein of the invention is supported by the fact that CD44v5 represents the most abundantly expressed CD44 variant in breast cancer (56% for CD44v5 vs. 24% for CD44v6 and 15% for CD44v8). Furthermore the expression of the CD44v5 variant is associated with a shorter survival time of breast cancer: The five year survival time is 71% in CD44v5 cancer patient versus 86% in CD44v5 negative patients (Tempfer et al. 1996).

In a preferred embodiment the CD44ex9-binding protein of the invention can be used for the diagnosis or treatment of colorectal cancer, as it was demonstrated that CD44v5 (and CD44v6) are associated with a poor prognosis in colorectal cancer. Hereby, patients with higher CD44v5 or CD44v6 content in tumor samples had a considerably shorter relapse-free survival (Vizoso et al. 2004).

In another preferred embodiment the CD44ex9-binding protein of the invention can be used for the diagnosis or treatment of head and neck squamous cell (HNSCC) tumors, as these tumors exhibit a high expression of CD44 and the subpopulation with high CD44 expression are less sensitive to radiation and chemotherapy (La Fleur et al, 2012).

In a further embodiment the CD44ex9-binding protein of the invention can be used for the diagnosis or treatment of melanomas, as a CD44-derived peptide showed in vitro and in vivo efficacy in a melanoma tumor model (Piotrowicz et al,. 2011).

In a preferred embodiment of the invention the CD44ex9-binding protein is used for preparing a contrast agent for medical use.

In a further preferred embodiment the contrast agent is capable to identify cancer cells or carcinoma in situ cells.

In a specific embodiment the binding protein or any derivatives, conjugates or fusion proteins is capable to identify cancer cells which are selected from the list consisting of adenocarcinoma cells, thymic epithelial tumor cells, cervical carcinoma cells, non-Hodgkin lymphoma cells, lung cell carcinoma cells, pancreas carcinoma cells and cancer stem cells.

It is disclosed hereby that the CD44ex9-binding fusion proteins or conjugates of the invention may be bound to nanoparticles of any kind. Several classes of nanoparticles are known in prior art and the skilled person can select the appropriate type of nanoparticle according to the specific therapeutic or diagnostic requirements.

Examples for nanoparticle to be coupled with the CD44ex9-binding protein are quantum dots, Noble metal clusters, superparamagnetic iron oxide nanoparticles (IONPs), block-copolymer micelles, nanocells, dendrimers, nanotubes, polymersomes, XPclad® nanoparticles, and nanoparticles consisting of amorphous silica surrounded by a crystalline luminescent calcium phosphate layer (e.g. ORMOBEAD®).

The ORMOBEAD particles can be suitably modified on the surface with polyethylene imine or TRIAMO yielding amine groups or 6-amino hexanoic acid (AHA) or with adipic acid yielding carboxyl groups. These groups can be used for the coupling to the proteins of the invention. The ORMOBEAD technology is disclosed by Dembski et al. (2013).

In one disclosure of the invention SiO₂/Zn₂SiO₄:Mn²⁺ and SiO₂/Ca₁₀(PO₄)₆OH:EU³⁺ core-shell nanoparticles with diameters below 100 nm are used as nanoparticles for coupling with the proteins of the invention. These particles are disclosed by Dembski et al. (2011a, 2011b).

In a further disclosure luminescent dye-labeled hybrid nanoparticles can be used. These nanoparticles consist of a SiO₂-based particle matrix with covalently attached organic fluorophores. They combine the optical properties of organic dye molecules and the inorganic particle matrix properties. As a result they show an increased resistance to photobleaching and a decreased dye leakage. Respective nanoparticles are disclosed by Probst et al. (2012).

In another disclosure, cadmium-free quantum dots can be used. These nanoparticles show bright emission in the visible and near infra-red region of the spectrum. Respective nanoparticles are developed by Nanoco Technologies Ltd. (Manchester, UK) and are disclosed in WO07/020416, WO08/100276, WO10/52455, WO10/15824, WO10/10329 and WO13/93631.

In one disclosure of the invention (group II-alloyed) group I-III-VI semiconductor quantum dots, group III-V quantum dots or micronized semiconductor nanocrystal complexes as developed by Evident Technologies (Troy, NY, USA) can be used. These nanoparticles are disclosed in WO07/118118, WO08/94292, WO06/17125 or WO05/110916 respectively.

In another disclosure superparamagnetic iron oxide nanoparticles (IONPs), block-copolymer micelles, nanocells, dendrimers, nanotubes, polymersomes and XPclad® nanoparticles can be used. Respective nanoparticles are disclosed by Singh and Lillard (2009) and Xie et al. (2010).

In a further disclosure of the invention non-Cd-nanoparticles can be used, comprising a core area being covered by a shell area which represents an antireflective coating of the core area. Respective nanoparticles are disclosed by US 2008/0286826 A1 (Philips Intellectual Property & Standards).

In another disclosure of the invention, magnetic particles can be used, which are especially suited for targeted drug delivery. In a preferred embodiment said magnetic particles consist of superparamagnetic metal oxides and/or metals and are coated with the peptides of the invention and optionally with one or more additional drugs. Respective magnetic particles are disclosed by EP 1 267 843 B1 (EUCRO).

The modified nanoparticles may preferably be employed as in vivo contrast agents for detecting CRC cells. WO 2007/057182 A3 discloses advantageous nanoparticles. Said nanoparticles are in particular those whose hydrodynamic diameter does not exceed 15 nm and which are non-inert in biological systems.

The nanoparticles as disclosed herein can be further conjugated to at least one tumor antigen-binding substance and/or cytotoxic agent.

In a disclosure of the invention the nanoparticle is further conjugated to a tumor antigen-binding substance which is selected from the list consisting of an antibody against CEA, an antibody against CA-19-9, and an adhesin or any combination thereof.

In a disclosure the adhesin conjugated to nanoparticle is modified in its amino acid sequence, and more preferably modified according WO 2009/106102 A1.

The nanoparticles as disclosed herein comprise at least three structures, namely an inorganic core which is coated by a layer comprising an imidazole component containing layer (which in the following is also referred to as a "passivation layer") which then in turn carries specific ligands, wherein said specific ligands may also be part of the layer. Said ligands result in the nanoparticle binding specifically to the target of the biological system.

In preferred nanoparticles, the inorganic core including the passivation layer surrounding it has a hydrodynamic diameter of no more than 15, if possible, preferably no more than 10 nm. Particular preference is given to hydrodynamic diameters of no more than 8 nm or no more than 5 nm. This applies especially to spherical nanoparticles. Nanoparticles of this size can be illuminated via the kidneys and therefore do not accumulate, or at most accumulate in tolerable quantities, in the body. This makes *in vivo* application possible. This applies in particular to nanoparticles having a hydrodynamic diameter of no more than 5 nm.

In an alternative disclosure, the nanoparticles may also be rod-like. In this disclosure, it is advantageous if the diameter of the rod does not exceed the abovementioned limit of 15 nm. Here too, preference is given to diameters in the range of 5, 8 or 10 nm to facilitate elimination from the body. Thus, for example, the nanoparticles employable may have length/breadth dimensions of 8 x 15 nm.

The nanoparticles employable as disclosed herein preferably have maximum emission at a wavelength between 600 and 700 nm, for example between 620 and 660, particularly preferably at about 625 nm or 655 nm. Said emission is readily visible to the human eye, and such nanoparticles can therefore be used directly as contrast agents for medical interventions. Consequently, auxiliary optical instruments may in some circumstances be dispensed with.

In an alternative disclosure, nanoparticles exceeding the abovementioned hydrodynamic diameters may be employed according to the disclosure, as long as the particles are guaranteed to be non-inert *in vivo.* The latter is the precondition for said particles to be biodegradable and, as a result, the metals (e.g. Cd) which are bound therein as particulates initially, to be converted into the ionic form. The degradation products can be illuminated via the kidneys.

Inorganic nanoparticles having a passivation layer containing an imidazole component are indeed non-inert *in vivo,* as has been demonstrated previously, but they are degraded under these conditions. Said nanoparticles therefore satisfy the criterion of biodegradability and of renal passage of the degradation products, which is particularly relevant for *in vivo* application. This was a surprise finding because the passivation layer serves especially also to increase the chemical and/or physical stability of the nanoparticles (in this context, see also the additional comments below). Thus, the relationship between on the one hand the stability of the nanoparticles which is required for good diagnostics, and on the other hand biodegradability which is required for renal passage of "large" particles is suitable for use as *in vivo* contrast agent.

The main task of the passivation layer is to increase fluorescence intensity and chemical and physical stability of the inorganic core. The inorganic cores coated by the passivation layer are characterized by a quantum yield of at least 10%, advantageously at least 30, 50 or even 70%. Quantum yield here means the ratio of the amount of the light emitted by a sample to the amount of light absorbed by the sample. Advantageously, the passivation layer has a thickness of no more than 1 nm. In this case, the diameter of the passivated core increased by no more than 2 nm.

Advantageously, the nanoparticles are in each case also provided with modifiers, in particular for improving compatibility with the biological environment. Preferably, the increase in the hydrodynamic radius due to the use of modifiers does not exceed 2 nm. In particular cases, the thickness of the passivation layer and the modifiers also depends on the relationships of the two structures among each other and in relation to the inorganic core.

The preferably used nanoparticles as disclosed herein, if restricted in size as mentioned above, are particularly suitable for the use as diagnostic agent in a living patient. Thus, the size reduction increases the rate of diffusion and depth of penetration into the tissue. This allows the nanoparticles to spread evenly and rapidly in the biological environment and also penetration as far as possible of a tissue (for example a tumor) after local administration. The nanoparticles as disclosed herein likewise allow systemic administration which may also be carried out by way of injection. However, local administration, for example topical application or intra- or peritumoral administration for the treatment of tumors is also possible.

Particularly advantageous disclosures of the invention comprising the nanoparticles coupled to the proteins of the invention have a hydrodynamic diameter of no more than 8, particularly preferably of no more than 4 nm. Nanoparticles of this order of magnitude may already be illuminated via the kidneys and therefore do not accumulate, or accumulate to a distinctly lesser extent, in the body. As a result, the nanoparticles of the invention reduce considerably the problem of long-term toxicity probably associated with the known quantum dots.

The nanoparticles advantageously emit a fluorescent spectrum between 600 and 700 nm, particularly preferably with maximum emission between 600 and 660 nm, particularly preferably between 620 and 660 nm. Said emission spectrum has the advantage of very high tissue transmission owing to only low absorption by hemoglobin and other light-absorbing substances in a living system (including water). Light of these wavelengths can still be sensed by the human eye and therefore enables the physician in charge of the treatment to identify the labeled tissue without any further complicated technical detection aids (e.g. CCD cameras). This is particularly advantageous when using the nanoparticles of the invention as contrast agents during surgical intervention for identifying CD44, CEA- and/or CA19-9-expressing cells, in particular for discriminating carcinogenic and healthy tissues.

In one disclosure, the preferably employable nanoparticles are known nanoparticles having a core of, for example, CdSe, CdS or CdTe, as described, for example, in US 2004/0247861 with reference to scientific publications. This printed publication also makes reference to documents regarding the preparation of the core materials, for example to US 6,179,912. A method of preparing nanoparticles is furthermore also disclosed in US 7,147,712 B2.

Particularly advantageously, the inorganic core of the nanoparticles essentially consists of semiconductors. These cores emit light of various colors, depending on their individual size and/or composition, but all of them absorb over a broad band within the same range of the light spectrum (UV to VIS range). Due to the high Stokes shift, excitation and emission spectra are far apart, enabling simple and simultaneous excitation of various nanoparticles. They have narrow and symmetric emission spectra which overlap only slightly or not at all. Other beneficial properties which are of great importance particularly for improved depth of filtration and *in vivo* labeling are the high quantum yield of up to 80% and high photostability.

Preferred nanoparticles have been disclosed, for example, in WO 2005/001889. Accordingly, they comprise an inorganic core made of an alloy of at least two semiconductors which either are distributed homogeneously or for which there is in each case a concentration gradient within the alloy. In respect of the disclosure of the nature and preparation of said nanoparticles, reference is made to WO 2005/001889 cited above. The cores may deviate in their size by in each case 5%.

Accordingly, the inorganic core of the nanoparticles may comprise an alloy of at least two semiconductors, wherein the core has a homogeneous composition and is characterized by a "band-gap energy" which is nonlinear to the molar ratio of the two semiconductors.

Alternatively, the core may be non-homogeneous, with the concentration of the first semiconductor gradually increasing, starting from the center of the core to the surface of the core, and the concentration of the second semiconductor gradually decreasing from the center of the core to its surface.

For both cores, at least one of the semiconductors is a group II-group VI semiconductor or a group III-group V semiconductor (the definition of groups corresponds to the groups of the Periodic Table of the Elements). For example, the alloy may be selected from the group of the following alloys: CdSeTe, CdSSe, CdSTe, ZnSeTe, ZnCdTe, CdHgS, CdHgTe, InGaAs, InGaP, GaAIAs, InGaN. These cores may moreover carry a coating of inorganic material such as, for example, semiconductors (e.g. ZnS). This additional layer is known to the skilled worker as "capping" or "shell".

Group II-group VI and group III-group V semiconductors are generally known and include, for example, CdS₁₋ₓSeₓ, CdS₁₋ₓTeₓ, CdSe₁₋ₓTeₓ, ZnSe₁₋ₓTeₓ, Zn₁₋ₓCdₓTe, Cd₁₋ₓHgₓS, Cd₁₋ₓHgₓTe, In₁₋ₓGaₓAs, Ga₁₋ₓAlₓAs and In₁₋ₓGaₓP. Preference is given to using the semiconductors CdSe₁₋ₓTeₓ, CdS₁₋ₓTeₓ, ZnSe₁₋ₓTeₓ, Zn₁₋ₓCdₓTe, Cd₁₋ₓHgₓS, Cd₁₋ₓHgₓTe, In₁₋ₓGaₓAs, In₁₋ₓGaₓP, where x is a fraction from 0 to 1.

The molar ratio of the semiconductors may be any molar ratio. However, if the alloy comprises CdSSe, preference is given to an alloy having the molecular formula CdS₁₋ₓSeₓ. If the alloy comprises CdSTe, preference is given to an alloy having the molecular formula CdS₁₋ₓTeₓ. If the alloy comprises ZnSeTe, preference is given to an alloy having the molecular formula ZnSe₁₋ₓTeₓ. If the alloy comprises ZnCdTe, preference is given to an alloy having the molecular formula of CdTe alone. In each of these cases, x is a fraction between 0 and 1.

These preferred inorganic cores of the nanoparticles may be prepared using the following steps: (i) preparation of a first solution under conditions which enable nanocrystals to form, (ii) preparation of a second solution which comprises a precursor of the semiconductors with a molar ratio under a condition which does not enable nanocrystals to form, (iii) addition of the second solution to the first solution which enables nanoparticles to form, and (iv) alteration of the conditions, which stops growth and formation of the nanocrystals. The method of preparing the cores is illustrated in WO 2005/001889.

In an alternative disclosure, the inorganic core may essentially consist of a noble metal cluster which preferably comprises 2 and 27 noble metal atoms. In a preferred embodiment, the noble metal was selected from a group consisting of gold, silver, copper, platinum, palladium, osmium, iridium, ruthenium and rhodium. The cluster may have varying charges.

These cores have the advantage that they can be detected readily as individual "nanodots", using a weak mercury lamp excitation, owing to their strong absorbance and emission. The nanoparticles as disclosed herein containing these cores can advantageously be used as fluorescent individual molecule label and mass label.

The term "noble metal" to a group of elements selected from a group consisting of gold, silver and copper, and the platinum group metals (PGM), platinum, palladium, osmium, iridium, ruthenium and rhodium. In preferred disclosure of the present invention, the noble metals are selected from the group consisting of gold, silver and copper. In a particularly preferred disclosure, the noble metal is silver or gold.

The term "cluster" relates to a compound of 2-27 atoms of a metal. Clusters are known inter alia from the fields of chemical catalysis, ceramics, semiconductor technology and material sciences. A person skilled in the art is therefore familiar with their preparation. WO 2004/003558 describes inter alia the preparation of noble metal clusters and in addition contains extensive further references on this subject. More specifically, it discloses the preparation of noble metal nanoclusters associated with organic molecules. The term association here means any form of binding, independently of the chemical or physical nature of the binding (thus, for example, covalent, non-covalent, electrostatic or van der Waals binding). Reference is made to WO 2004/003558 in respect of preparation of the nanoclusters as core of the nanoparticles as disclosed herein.

The nanoparticles preferably employable according to the disclosure have a passivation layer which increases fluorescence intensity and improves the chemical and physical stability of the inorganic core. As a result, the nanoparticles emit light preferably with a quantum yield of more than 10%, preferably of more than 50%.

Said nanoparticles preferably have a storage stability of at least 12 months in an aqueous environment at 4°C and are, if possible, stable across a pH range from pH 5 to pH 10, preferably from pH 7 to pH 10, i.e. they exhibit deviations of less than 50% in respect of their specific spectral characteristics such as quantum yield, position of maximum emission, half-width of the emission spectrum. Preferred particles exhibit deviations of less than 10% in respect of these specific spectral characteristics.

The nanoparticles employable according to another disclosure of the invention exhibit essentially a constancy/stability of the properties of the core (including the passivation layer surrounding it) also under biological (i.e. physiological) conditions or *in vivo* over a period of at least three days. Preferred particles exhibit a constancy/stability of this kind for a period of from 7 to 14 days, wherein by way of stability retaining at least 50% of the one constancy of the properties. This information refers especially to the stability of the nanoparticles in the actual target organ. It is noteworthy that the stability of the nanoparticles in organs which have primarily catabolic function may be distinctly less stable (for example in the liver). This may even be expressly desirable.

Although the nanoparticles are stable in the above sense, they are nevertheless fundamentally degradable *in vivo* and consequently are non-inert. In this sense, "non-inert" means that at least 50% of the nanoparticles have already been degraded after 12 weeks or more post-administration. Preference is given to at least 50% degradation being detectable already after 8, 6 or 4 weeks. Detection of the particles remaining in the body includes detection in body organs and in the plasma for this purpose. Accordingly, "inert" means that more than 50%, even up to nearly 100%, of the particles are still detectable in the body of the patient after 4 weeks post-administration.

Degradability of the nanoparticles can be detected by assays which are known to the skilled worker, namely, for example, by inductively coupled plasma mass spectrometry (ICP-MS), which assays may also be supplemented by fluorescence spectrometry measurements, if the samples are suitable.

The passivation layer contains at least one imidazole component. Such a compound is capable of coordinating metal atoms or metal ions, for example zinc ions, mercury ions or cadmium ions. In a preferred embodiment, the imidazole group is in a terminal position based on the structure of the molecule. The passivation layer may furthermore have a crosslinker, or the cyclic or linear imidazole component may also act as a crosslinker. The crosslinker may be alkaline.

The coordination compounds containing metal atoms or metal ions may functionally bind to fluorescent inorganic cores by means of chelation, coordination or electron donor properties of Lewis bases and have correspondingly conjugated moieties/groups. Said molecules may moreover contain moieties which impart solubility or wettability to the cores coated with them in aqueous solutions.

The imidazole component is suitably crosslinked by a phosphine compound, being preferably an alkylphosphine compound.

The term "imidazole component" means for the purposes of the present description a heterocyclic or heteroaromatic molecule which contains at least one imidazole group (including imidazole derivatives) and which is available for binding of the inorganic core or the passivation layer having a metal such as cadmium, zinc, gallium, or a metal cation or a substrate containing such a cation. In this connection, preferably at least one imidazole group should be at a terminal position based on the structure of the molecule. The imidazole component in its functional form binds via the ring which contains delocalized molecular orbitals to the fluorescent nanocrystal. Usually, the nitrogen atoms of the imidazole ring serve as coordination ligands to functionally bind a metal ion such as cadmium or zinc.

In one embodiment, the imidazole component comprises reactive functional groups such as one or two amino acid(s), for example histidine, carnosine, anserine, baleine, homocarnosine, histidylphenylalanine, cyclo-histidylphenylalanine, 5-amino-4-imidazole-carboxamide, histidylleucine, 2-mercaptoimidazole, boc-histidine, hydrazide, histinol, 1-methylhistidine, 3-methylhistidine, imidazolysine, imidazole-containing ornithine (e.g. 5-methylimidazole), imidazole-containing alanine (e.g. (beta)-(2-imidazolyl)-L-(alpha) alanine), carzinine, histamine. These histidine-based molecules or imidazole-containing amino acids may be synthesized by generally known methods.

The term "phosphine" means for the purpose of the present disclosure a molecule which has at least one phosphine group (including their derivatives) for binding or chelating a nonmetal such as Se, S or other nonmetals or substrates containing such atoms, and which provides at least one functional group (for example hydroxyl-, amino-, thiol-, carboxyl-, carboxamide-etc.) for reaction with neighboring molecules.

In a preferred disclosure of the invention the imidazole component is a peptide comprising at least one histidyl residue, and preferably a dipeptide containing one or two His-residues.

In a further preferred disclosure the imidazole component is a mixture of different histidyl-containing dipeptides.

Preferably, at least one phosphine group should be located at a terminal position based on the structure of the molecule. The phosphine moieties serve as coordination ligands to bind in its functional form with a fluorescent core or a compound from the shielding layer a nonmetal or ion such as Se or S.

In a preferred disclosure, the phosphine-containing compound includes one, two or more phosphine groups coupled to one another (e.g. in polymeric form) which may include hydroxymethylphosphine compounds or the like but without being limited thereto. Phosphine-containing compounds may be synthesized by generally known methods. Furthermore, alkylphosphine-containing compounds are known to possibly also have one or more additional functional groups (e.g. hydroxyl-, amino-, thiol-, carboxyl-, carboxamide-, etc.). Examples of derivatives are hydroxymethylphosphine derivatives, amides or esters, as long as said derivatization is compatible with the functions described herein of phosphine as coating.

Particular preference is given to tris(hydroxymethyl)phosphine and β-[tris(hydroxymethyl)phosphino]propanoic acid for coating the fluorescent inorganic cores of the nanoparticles as disclosed herein. Crosslinked phosphine-containing compounds are well known to additionally be able to functionally bind to metal atoms and/or ions such as Zn or Cd. Isocyanates or alkylcyanoacrylates functionalized in this respect may furthermore be useful as crosslinkers for ligands and the formation of adducts with fluorescent cores. Said crosslinkers may also be basic.

The passivating effect of the passivation layer present according to the present disclosure is based on the shielding of surface cadmium or zinc atoms or the like by complex formation with the imidazole component, and on the shielding of the counteratoms (Se or S or the like) via complex formation with the phosphine-containing compounds.

The passivation layer of the nanoparticles as disclosed herein has been disclosed in US 2004/0247861 A1. This laid-open application describes the preparation of inorganic cores coated with the passivation layer, for example of quantum dots. Reference is therefore made to US 2004/0247861 for purposes of disclosure of the preparation of the passivation layer employed according to the dislcosure and of the inorganic cores coated therewith.

The molecules of the passivation layer may furthermore have or carry chemical groups in order to bind and crosslink target molecules and cells (specific ligands). In the presence of appropriately suitable reagents such as ZnSO₄ and Na₂S, said molecules or compounds may form a passivation layer with the molecules on the fluorescent core ("capping" or "shell"). These reagents may also functionally bind to atoms or ions on the surface of the fluorescent nanocrystal and, as a result, this additional passivation layer may also be formed directly on the surface of the core.

In an advantageous disclosure, the nanoparticles of the invention may additionally have modifiers which may consist of organic and/or inorganic moieties. They are used for improving compatibility, efficacy and/or solubility of the nanoparticles in a liquid or a suspension medium, in particular in the physiological environment. This surface modification is especially advantageous for achieving very low unspecific adsorption and increased compatibility in biological systems, in particular in the human body.

One possibility is to modify the surface with polyethylene glycol (PEG) which has already been approved for particular medical applications, in particular in low molecular weight forms for the nanoparticle to maintain a small overall size. Thereby both biocompatibility and blood circulation time of the nanoparticles and also the efficiency of uptake into cells may be increased. Combining a low molecular weight PEG layer with other substances such as vitamins, for example folic acid, may achieve a lower uptake of said nanoparticles into macrophages because protein adsorption to the nanoparticles, which is reduced thereby, makes recognition of said nanoparticles by the immune system more difficult.

Another possible advantageous surface modification by using modifiers is the coating with monosaccharides, di- or trisaccharides at up to low molecular weight polysaccharides composed of one type of monosaccharide or different monosaccharides. One possible type of development is a modification with polyglucose, for example, in which dextran can be used which has been proved medically as blood substitute. It exhibits good biocompatibility/ tolerance. Another embodiment is the use of stereoisomeric forms (D-/L-) of saccharides in order to counteract possible degradation.

Another embodiment is the use of biologically compatible hydrophilic vitamins as modifiers, for example thiamine, riboflavin, niacin, pyridoxine, cobalamin, panthothenic acid, ascorbic acid and folic acid. Thus, for example, folic acid can lead to a preferred binding of nanoparticles to cancer cells. This vitamin exhibits only low immunogenicity and therefore high biocompatibility. Internalization of the nanoparticles is facilitated by binding to the membrane-bound folic acid receptor.

Surface modifications are also possible with lipophilic vitamins such as retinol, cholecalciferol, tocopherol and phylloquinone. Thus, for example, vitamin E can increase the cellular uptake of nanoparticles.

Fatty acids such as, for example, 1-octadecene or 18-methyleicosanoic acid and their derivatives, may increase solubility and stability of the colloids and have a terminal functional carboxyl group which may be utilized for subsequent binding of specific ligands. It is therefore useful to include fatty acids as modifiers.

Another embodiment of surface modification is a coating with polyalcohols such as, for example, diethylene glycol (DEG), which are particularly good at reducing unspecific protein adsorption. The same applies to polytetrafluoroethylene (PTFE, Teflon), in particular in its low molecular weight forms, which can achieve reduced protein adsorption. Polytetrafluoroethylene is frequently used in cardiosurgical applications.

Surface modifications can likewise be carried out using one or more naturally occurring amino acids which include both proteinogenic and non-proteinogenic amino acids, and synthetic amino acids. Both stereoisomers (D- and L-forms) may be used here. Di-, tri-, tetra-up to small polypeptides of the abovementioned amino acids hardly stimulate the immune system and are therefore likewise suitable for a thin compatibility layer. They may be artificial amino acid sequences as well as sequences from biological proteins. Peptide derivatives of natural proteins such as, for example, phytochelatin, may likewise be used for surface modification. Surface modification with Tat peptide and Tat peptide-containing peptides is another possibility of making nanoparticles available for the use in biomedical applications. The Tat peptide is an effective molecule, for example, for delivering gold nanoparticles through the cell membrane all the way into the nucleus.

Another disclosure of possible modifiers is the formation of a phosphorylcholine coating. Phosphorylcholine reduces a possible unspecific protein adsorption, for example on contact lenses. Owing to its non-thrombogenic properties, a phosphorylcholine modification can readily be employed in biological systems and is distinguished by high storage stability.

Since polylactate is biocompatible, this substance is used in a variety of medical applications. More specifically, low molecular weight forms of polylactate constitute another possible surface modification of the nanoparticles as disclosed herein. Both stereoisomers (D-/L-forms) may be employed here in order to reduce possible biodegradation.

Apart from the surface modifications mentioned, proteolytically cleavable binding of unspecific proteins to the nanoparticles is also possible. This may increase biocompatibility/compatibility. At the target location, the large protein may be removed with the small nanoparticles being released in the tissue. Said removal may also take place after an appropriate dwell time. Suitable for this are preferably commonly used proteins such as, for example, transferrin, lactoferrin, ceruloplasmin, elastin and albumin in addition to other proteins which reduce unspecific adsorption. Thus, for example, surface coating composed of combinations of polypeptides with elastin may prevent undesired clot formation and therefore increase the biocompatibility of the nanoparticles.

The major serum protein albumin may reduce non-specific interactions with plasma membranes. Furthermore, the appropriately modified nanoparticle retains the ability to develop specific interactions with target cells by a specific ligand simultaneously binding to the particle surface. A coating with serum albumin may result in a substantially longer blood circulation time by preventing a rapid uptake by microphages after intravenous administration, than is the case with uncoated nanoparticles.

The combination of reduced hydrodynamic diameter which results in the higher rates of diffusion and perfusion mentioned, together with the properties and improvements described above and the high fluorescence intensity, especially in the visible red light range, renders the nanoparticles as disclosed herein a simple diagnostic agent which can be employed in many different ways for selective and accurate discrimination of tissue forms *in vivo.* These possibilities, in combination with antigen-specific biomarkers, are used especially for identifying CD44v5, CEA- and/or CA-19-9-expressing cells, in particular for distinguishing abnormal, (pre-)carcinogenic tissue from normal tissue, assisting in the visual assessment during surgical intervention for a more precise tumor resection.

The disclosed nanoparticles employable herein therefore serve as contrast agents. This applies in particular to the use in cancer diagnosis and surgery.

The nanoparticles carrying the CD44ex9-binding protein according as disclosed herein may be employed either as *in vitro,* as *ex vivo* or as *in vivo* diagnostic agent, theranostic agent and/or therapeutic agent. For this purpose, they may be administered locally (e.g. intratumorally, intramuscularly or into surgically accessible tissues/organs) or else also systemically (e.g. intravenously). Local/topical administration may be provided for by way of a liquid, spraying solution, foam, cream or active patch. This may be preferred in particular for the treatment/diagnosis of hollow organs such as in the case of bowel cancer. Oral intake is also possible, for example as syrup or in the form of tablets or capsules. Inhalation is equally possible (e.g. spray). Anal administration by suppository is envisaged. In one variant, the nanoparticles may be implanted in depot form.

In a preferred embodiment of the invention the CD44ex9-binding protein may be used for targeted drug delivery. In the context of the present invention, targeted drug delivery is defined as a measure to concentrate the medication in the tissues of interest while reducing the relative concentration of the medication in the remaining tissues. For this purpose, the CD44ex9-binding protein alone or as a fusion protein or conjugate is bound to a drug delivery vehicle. There are numerous types of drug delivery vehicles which are known to the skilled person and which he can select according the specific purpose, such as, e.g. polymeric micelles, liposomes, lipoprotein-based drug carriers, nano-particle drug carriers, dendrimers etc. An ideal drug delivery vehicle must be non-toxic, biocompatible, non-immunogenic, biodegradable and avoids recognition by the host's defense mechanisms.

In a preferred embodiment of the invention liposomes comprising lipid-derivatized bisphosphonic acids can be used for targeted drug delivery, especially for a delivery to bone structures. Respective lipid-derivatized bisphosphonic acids are disclosed by WO 2005/070952 A2 (MCS Micro Carrier Systems GmbH).

In a preferred embodiment of the invention the CD44ex9-binding protein of the invention may be used as *ex vivo* or *in vitro* diagnostic agent. The in vitro or ex vivo diagnostic use relates to the detection of the CD44v5 protein in a sample derived from a patient. The sample to be analysed can be of any type including tissues, organs and biopsies therefrom or body fluids such as blood, serum, urine, saliva or cerebral liquor.

For the diagnostic use, the CD44ex9-binding protein is labeled for detection. Appropriate labels are known in prior art and include dyes such as luminescent or fluorescent dyes, a radioactive isotope, an enzyme, a protein tag such as a FLAG tag, a GST-tag, an MBP-TAG or a His-tag, or a nanoparticle.

For the incorporation of a label several prior art methods are known which rely on the random modification of sulfhydryl, amine, or carboxyl groups. Alternatively, genetic engineering can be used to introduce or remove additional reactive groups, such as a cysteine residue.

As an alternative method a short peptide tag can be attached to the protein that functions as a label acceptor site. Here the label is attached either through an intrinsic activity of the tag or the activity of an enzyme supplied in trans. Examples for said activity-associated labeling include site-specific biotinylation, as mediated by the biotin holoenzyme synthetase BirA which allows tagged proteins to be combined with a broad range of avidin/streptavidin reagents. Proteins fused to the LUMIO-tag (a hexapeptide containing a tetracysteine motif) can be stained directly with dyes containing two arsenic atoms.

In a preferred embodiment of the invention, the engineered version of the human DNA-repair enzyme *O*(6)-alkylguanine DNA alkyl transferase (AGT; also known as SNAP-Tag) is fused to the CD44ex-binding protein. Substrates containing *O*(6)-benzylguanine are covalently bound to the fusion proteins via a stable thioether bond in a rapid and highly specific self-labeling reaction. This label technique and its use for diagnostic purposes is disclosed by Kampmeier et al. (2009) and Xie et al. (2010).

In one disclosure , the CD44ex9-binding protein is bound to a nanoparticle. Various types of nanoparticles are known in prior art and also disclosed in the present application.

In a further preferred embodiment, the labeled CD44ex9-binding protein is contained within an aqueous immersion bath. By dipping the sample into the immersion bath, the labeled CD44ex9-binding protein will specifically label the CD44v5 proteins of the sample which can be directly assessed visually by the investigator. This procedure is especially suited for surgery, whereby the withdrawn tissue can be analysed during surgery for the presence of tumor markers. As a result the surgeon has an immediate feedback regarding the precision of tumor resection.

The term "diagnostic agent" is used in the context of the present invention as a synonym for "contrast agent", i.e. it serves for the discriminating visualization of morphological or functional structures in biological systems, especially in living people, to assist a medical intervention.

The nanoparticles as disclosed herein may be employed as diagnostic agent especially in surgical interventions. They can likewise be used in minimally invasive methods (e.g. endoscopy, laparoscopy). Combination with imaging methods such as PET, MRT, CT, etc. is worthwhile.

As already stated above, the use in the form of local administration is particularly advantageous. The amount of Cd employed on local administration in this connection advantageously does not exceed one tenth of the total exposure which normally accumulate anyway during the course of life in the liver and kidney of a person of advanced age and usual lifestyle. The total exposure of these organs is about 18 mg. Accordingly, it is advantageous on local administration for the amount of nanoparticles to be limited so that the amount of Cd supplied at least does not substantially exceed 2 mg. In a particularly preferred disclosure, tumor visualization is possible even with an amount of contrast agent which does not exceed a total amount of 0.6 mg, particularly preferably 0.2 mg, of cadmium.

"Local administration" means for the purpose of the invention any administration, on which an increased amount or dose of the contrast agent can be expected in distinct regions of the body depending on the manner of administration. Accordingly a vascular administration of the contrast agent is also a local administration, if accompanying measures by the administering personnel, such as, for example, applying a ligature to afferent or efferent vessels, prevent the contrast agent from spreading across the blood vascular system in the body in an essentially unimpeded manner.

The particular advantage of this disclosure is that the use of the nanoparticles in medical application on a living person is thereby possible because otherwise - i.e. as systemic administration - this is precluded because of the toxicity associated therewith. This is because local administration reduces the dose of nanoparticles necessary for adequate visualization.

It has emerged that the Cd-containing contrast agent is advantageously employed according to the present disclosure for visualizing a tumor *in vivo* in a dose corresponding to an amount of from 0.002 to 0.02 mg of Cd per cm³ of tumor tissue. Dosages of the contrast agent of from 0.002 to 0.015 mg of Cd/cm³ of tumor tissue are particularly advantageous, in particular those between 0.002 and 0.010 mg of Cd/cm³. It is possible with this advantageous dosage to visualize tumors with a volume of up to about 150 cm³ *in vivo* without thereby exceeding the normally acceptable upper limit of exposure for humans. The visualization of tumors with a volume of up to 50 cm³ is particularly favorable.

The investigations may relate to all accessible tissues/organs of the patient, especially in the skin, hollow organs (e.g. in the gastrointestinal, urogenital, respiratory tract) or else externally accessible regions of the sensory organs and also the cardiovascular system.

Use as an *in vitro* diagnostic agent is also possible, for example immunohistochemistry or FACS, and ELISA. A combination of *in vivo* and *in vitro* diagnosis (e.g. biopsy material) is particularly advantageous.

The CD44ex9-binding proteins according to the invention may remain bound to the nanoparticles or may be removable or detectable or releasable.

In a further aspect the application discloses CD44 v5-peptides as listed in the following table and designated as SEQ ID No. 11 to 38. These peptides are 12-mers with overlapping sequences covering the complete v5 domain of human CD44.

| CD44v5-Peptide No. | Amino acid sequence | SEQ ID No. |
|---|---|---|
| 1 | DVDRNGTTAYEG | 11 |
| 2 | VDRNGTTAYEGN | 12 |
| 3 | DRNGTTAYEGNW | 13 |
| 4 | RNGTTAYEGNWN | 14 |
| 5 | NGTTAYEGNWNP | 15 |
| 6 | GTTAYEGNWNPE | 16 |
| 7 | TTAYEGNWNPEA | 17 |
| 8 | TAYEGNWNPEAH | 18 |
| 9 | AYEGNWNPEAHP | 19 |
| 10 | YEGNWNPEAHPP | 20 |
| 11 | EGNWNPEAHPPL | 21 |
| 12 | GNWNPEAHPPLI | 22 |
| 13 | NWNPEAHPPLIH | 23 |
| 14 | WNPEAHPPLIHH | 24 |
| 15 | NPEAHPPLIHHE | 25 |
| 16 | PEAHPPLIHHEH | 26 |
| 17 | EAHPPLIHHEHH | 27 |
| 18 | AHPPLIHHEHHE | 28 |
| 19 | HPPLIHHEHHEE | 29 |
| 20 | PPLIHHEHHEEE | 30 |
| 21 | PLIHHEHHEEEE | 31 |
| 22 | LIHHEHHEEEET | 32 |
| 23 | IHHEHHEEEETP | 33 |
| 24 | HHEHHEEEETPH | 34 |
| 25 | HEHHEEEETPHS | 35 |
| 26 | EHHEEEETPHST | 36 |
| 27 | HHEEEETPHSTS | 37 |
| 28 | HEEEETPHSTST | 38 |

In one aspect, the application discloses a peptide as designated as SEQ ID No. 11 to 38 having an N-terminal and/or C-terminal deletion of one, two or three amino acids.

In another aspect, the application discloses a peptide as designated as SEQ ID No. 11 to 38 having an N-terminal and/or C-terminal extension of one, two or three amino acids, whereby these additional amino acids are those of the respective CD44v5 amino acid sequence.

As disclosed herein, said peptide can be used for blocking the CD44ex9-binding protein of the invention. Due to this antagonistic activity, these peptides could be used to verify the specificity of an *in vitro, ex vivo* or *in vivo* CD44 labelling.

Furthermore, said peptide could be used as antagonist for the therapeutic use of the CD44ex9-binding protein. In this context the peptide could be used to regulate, diminish or cancel the effect of the CD44ex9-associated therapy.

In a therapeutic setting the CD44v5 peptides of the invention could directly bind to the endogenous ligands of the CD44 receptor. This could lead to neutralization of these ligands (making them unavailable for CD44 receptors) and could also lead to a rapid clearance by macrophages.

As disclosed herein, one or more of these peptides according SEQ ID No. 11 to 38 could be used as a vaccine in order to stimulate the immune system of the individual to develop adaptive immunity against the CD44 variants expressing the v5-domain. As a vaccine said peptides could be used for prevention of cancer or for an immunotherapy of cancer.

Due to successful outcome of the two hybrid screening leading to the identification of the CD44ex9-binding protein, the respective peptides have been validated as immunologically relevant epitopes. Hence, they are suitable for the use as vaccines.

The application discloses a vaccine composition comprising an isolated protein according SEQ ID No. 11 to 38 or a peptide fragment hereof or a nucleic acid encoding said protein or said peptide fragment for use as a medicament in the prevention or treatment of a cancer.

In a further aspect the invention relates to a pharmaceutical composition comprising the CD44ex9-binding protein of the invention or a labelled CD44ex9-binding protein, which preferably is bound to a nanoparticle, and a pharmaceutically acceptable carrier.

As the peptides of the invention are relatively small molecules it may be required in such compositions to combine the peptides with various materials such as adjuvants, to produce vaccines, immunogenic compositions, etc. Adjuvants, broadly defined, are substances which promote immune responses. Frequently, the adjuvant of choice is Freund's complete or incomplete adjuvant, or killed B. pertussis organisms, used e.g. in combination with an alum-precipitated antigen. A general discussion of adjuvants is provided in Goding, Monoclonal Antibodies: Principles & Practice (2nd edition, 1986) at pages 61-63. According to Goding, a coupling to an immunogenic carrier is recommended when the antigen of interest is of low molecular weight or is poorly immunogenic. Examples of such carrier molecules include keyhole limpet haemocyanin, bovine serum albumin, ovalbumin and fowl immunoglobulin. Various saponin extracts have also been suggested to be useful as adjuvants in immunogenic compositions. Recently, it has been proposed to use granulocyte-macrophage colony stimulating factor (GM-CSF), a well-known cytokine, as an adjuvant (WO 97/28816).

The vaccine compositions preferably comprise an adjuvant and/or a carrier. Examples of useful adjuvants and carriers are given herein below.

Thus the CD44v5-peptide or peptide fragment thereof present in the composition can be associated with a carrier such as e.g. a protein or an antigen presenting cell such as e.g. a dendritic cell (DC) capable of presenting the CD44v5-peptide or fragment thereof to a T-cell.

Adjuvants are any substance whose admixture into the vaccine composition increases or otherwise modifies the immune response to the CD44v5 protein. Carriers are scaffold structures, for example a polypeptide or a polysaccharide, to which the CD44v5 peptide or fragment thereof is capable of being associated.

Adjuvants could for example be selected from the group consisting of: AlK(SO₄)₂, AlNa(SO₄)₂ AlNH₄(SO₄), silica, alum, Al(OH)₃, Ca₃(PO₄)₂, kaolin, carbon, aluminium hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MOP),N-acetylmuramyul-L-alanyl-D-isoglutaminyl-L-alanine-2-(1'2'-dipalmitoyl-sn-glycero-3-hydroxphosphoryloxy)-ethylamine (CGP 19835A, also referred to as MTP-PE), RIBI (MPL+TDM+CWS) in a 2% squalene/Tween-80-RTM-emulsion, lipopolysaccharides and its various derivatives, including lipid A, Freund's Complete Adjuvant (FCA), Freund's Incomplete Adjuvants, Merck Adjuvant 65, polynucleotides (for example, poly IC and poly AU acids), wax 0 from Mycobacterium, tuberculosis, substances found in Corynebacterium parvum, Bordetella pertussis, and members of the genus Brucella, liposomes or other lipid emulsions, Titermax, ISCOMS, Quil A, ALUN (see US 58767 and 5,554,372), Lipid A derivatives, choleratoxin derivatives, HSP derivatives, LPS derivatives, synthetic peptide matrixes or GMDP, Interleukin 1, Interleukin 2, Montanide ISA-51 and QS-21. Preferred adjuvants to be used with the disclosure include oil/surfactant based adjuvants such as Montanide adjuvants (available from Seppic, Belgium), preferably Montanide ISA-51. Other preferred adjuvants are bacterial DNA based adjuvants, such as adjuvants including CpG oligonucleotide sequences. Yet other preferred adjuvants are viral dsRNA based adjuvants, such as poly I:C imidazochinilines are yet another example of preferred adjuvants. In addition, preferred adjuvants are liposomes. The most preferred adjuvants are adjuvants suitable for human use.

Montanide adjuvants (all available from Seppic, Belgium), may be selected from the group consisting of Montanide ISA-51, Montanide ISA-50, Montanide ISA-70, Montanide ISA-206, Montanide ISA-25, Montanide ISA-720, Montanide ISA-708, Montanide ISA-763A, Montanide ISA-207, Montanide ISA-264 , Montanide ISA-27, Montanide ISA-35, Montanide ISA 51 F, Montanide ISA 0160 and Montanide IMS, preferably from the group consisting of Montanide ISA-51, Montanide IMS and Montanide ISA-720, more preferably from the group consisting of Montanide ISA-51. Montanide ISA-51 (Seppic, Inc.) is oil/surfactant based adjuvants in which different surfactants are combined with a non-metabolizable mineral oil, a metabolizable oil, or a mixture of the two. They are prepared for use as an emulsion with an aqueous solution comprising the CD44v5-peptide or a fragment thereof. The surfactant is mannide oleate. OS-21 (Antigenics; Aquila Biopharmaceuticals, Framingham, MA) is a highly purified, water-soluble saponin that handles as an aqueous solution. OS-21 and Montanide ISA-51 adjuvants can be provided in sterile, single-use vials.

A vaccine composition as disclosed herein may comprise more than one different adjuvant. Furthermore, the application discloses a therapeutic composition further comprising any adjuvant substance including any of the above or combinations thereof. It is also contemplated that the CD44v5 peptide or fragments thereof, and the adjuvant can be administered separately in any appropriate sequence.

A carrier may be present independently of an adjuvant. The function of a carrier can for example be to increase the molecular weight of the peptide or the fragment thereof in order to increase their activity or immunogenicity, to confer stability, to increase the biological activity, or to increase serum half-life. Furthermore, a carrier may aid presenting the CD44v5 peptide or fragments thereof to T-cells. The carrier may be any suitable carrier known to the person skilled in the art, for example a protein or an antigen presenting cell. A carrier protein could be but is not limited to keyhole limpet haemocyanin, serum proteins such as transferrin, bovine serum albumin, human serum albumin, thyroglobulin or ovalbumin, immunoglobulins, or hormones, such as insulin or palmitic acid. For immunization of humans, the carrier must be a physiologically acceptable carrier acceptable to humans and safe. However, tetanus toxoid and/or diphtheria toxoid are suitable carriers in one disclosure of the application. Alternatively, the carrier may be a dextran for example sepharose.

Accordingly, the application discloses a therapeutic composition further comprising an adjuvant substance including any of the above or combinations thereof. It is also contemplated that the antigen, i.e. the peptide and the adjuvant can be administered simultaneously or separately in any appropriate sequence.

The pharmaceutical compositions prepared according to the invention comprise the CD44ex9-binding protein peptide together with a pharmaceutically acceptable carrier. The term "pharmaceutically acceptable" includes any carrier which does not interfere with the effectiveness of the biological activity of the CD44ex9-binding protein and which is not toxic to the host to which it is administered.

As one embodiment of the invention, the compositions are prepared using CD44ex9-binding protein stabilized with human serum albumin. For this purpose, a preparation of CD44ex9-binding protein is lyophilised with human serum albumin in vials.

In certain embodiments, the pharmaceutical composition further comprises one or more other drugs that act as toxin, cytokine, cytostatic agent, or immunomodulatory agent.

As used herein, the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils or other synthetic solvents; antibacterial agents such as benzyl alcohol or methyl paraben; antioxidants such as ascorbic acid or sodium bisulfate; chelating agents such as EDTA; buffers such as acetates, citrates or phosphates and agents for the adjustment of tonicity such as sodium chloride or dextrose. The pH value can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions or dispersions and sterile powders for the extemporaneous preparation of sterile injectable solutions or dispersion. For intravenous administration, suitable carriers include physiological saline, bacteriostatic water, Cremophor ELTM (BASF, Ludwigshafen, BRD) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, and the like), and suitable mixtures thereof. The proper fluidity can be maintained, for example, by the use of a coating such as lecithin, by the maintenance of the required particle size in the case of dispersion and by the use of surfactants.

Prevention of the action of microorganisms can be achieved by various antibacterial and antifungal agents, for example, parabens, chlorobutanol, phenol, ascorbic acid, thiomersal, and the like. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminium monostearate.

Sterile injectable solutions can be prepared by incorporating the active compound (e.g., the CD44ex9-binding protein) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatine capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose; an excipient such as starch or lactose; a disintegrating agent such as alginic acid, sodium starch glycollate (e.g. Primogel ®), or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

For administration by inhalation, the compounds are delivered in the form of an aerosol spray from a pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the bioactive compounds are formulated into ointments, salves, or creams as generally known in the art.

The composition can also be prepared in the form of suppositories (e.g., with conventional suppository bases such as cocoa butter and other glycerides) or retention enemas for rectal delivery.

In one embodiment, the therapeutic moieties, which may contain the CD44ex9-binding protein, are prepared with carriers that will protect the compound against rapid elimination from the body, such as a controlled release formulation, including implants and microencapsulated delivery systems. Methods for preparation of such formulations will be apparent to those skilled in the art. The materials can also be obtained commercially from e.g. Alza Corporation and Nova Pharmaceuticals Incorporation. Liposomal suspensions (including liposomes targeted to cancer cells with antibodies to tumor antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form, as used herein, includes physically discrete units suited as unitary dosages for the subject to be treated; each unit contains a predetermined quantity of active protein calculated to produce the desired therapeutic effect in association with the required pharmaceutical carrier. The specification for the dosage unit forms of the invention are dictated by and directly dependent on the unique characteristics of the active protein and the particular therapeutic effect to be achieved, and the limitations inherent in the art of compounding such an active protein for the treatment of individuals.

Toxicity and therapeutic efficacy of the protein of the invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Proteins which exhibit large therapeutic indices are preferred. While proteins that exhibit toxic side effects may be used, care should be taken to design a delivery system that targets such proteins to the site of affected tissue in order to minimize potential damage to non-cancer cells and, thereby, reduce side effects.

The data obtained from the cell culture assays and animal studies can be used in formulating a range of dosage for use in humans. The dosage of such compounds lies preferably within a range of circulating concentrations that includes the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (f.e., the concentration of the test compound which achieves a half maximal inhibition of symptoms) as determined in cell culture. Such information can be used to more accurately determine useful doses in humans. Levels in plasma may be measured, for example, by high performance liquid chromatography.

The pharmaceutical compositions can be included in a container, pack, or dispenser together with instructions for administration.

In another aspect, the invention provides a medicament/dosimeter combination package comprising: a) a medicament to be individually dosed, and b) a diagnostic indicator system for a patient-specific property that is relevant for the action, side effect, interaction, metabolism, absorption, distribution, metabolism, and elimination of the medicament to be administered to a patient, wherein the patient-specific property is selected from the group consisting of an endogenous substance, a regulation mechanism, a gene or an indication system. In the above described combination package, the medicament or the diagnostic indicator system can be the isolated CD44ex9-binding protein or any fusion protein or conjugate therewith.

A suitable combination package is disclosed by EP 1 542 644 B1 (MCS Micro Carrier Systems GmbH) relating to preparation and use of said medicament/dosimeter combination.

Another aspect of invention includes methods for preparing pharmaceutical compositions for modulating the expression or activity of the protein of the present invention. Such methods comprise formulating a pharmaceutically acceptable carrier with an agent which modulates expression or activity of the protein of the present invention. Such compositions can further include additional active agents. Thus, the invention further includes methods for preparing a pharmaceutical composition by formulating a pharmaceutically acceptable carrier with an agent which modulates expression or activity of the protein of the present invention and one or more additional bioactive agents.

### Definitions

In the context of the present invention, the term "CD44ex9-binding protein" is used for a protein with a length of 300 amino acids or less that binds to the polypeptide encoded by exon 9 of the human CD44 gene. In the description it is also referred to as "binding protein" or "active compound".

A protein in the context of the invention is defined as a molecule that is composed of amino acids. The term protein therefore includes dipeptides, tripeptides, tetrapeptides pentapeptides, hexapeptides, heptapeptides, octapeptides, longer oligopeptides and amino acid chains. The amino acid chain can be in a linear or a branched form.

Amino acids in the context of the present invention are organic compounds that contain at least one amino and at least one carboxyl group which in the protein form an amide bond.

The amino acids that are part of the protein can be taken from the 20 standard alpha-amino acids or non-standard amino acids, like selenocysteine, pyrrolysine, lanthionine, hydroxyproline, carboxygluatamate, 2-aminoisobutyric acid, dehydroalanine. Furthermore, other amino acids like beta-amino acids (f.e. beta alanine), gamma amino acids or even higher amino acids could be part of the protein.

For certain amino acids different enantiomeric or stereoisomeric form could be chosen, like e.g. for the alpha amino acids that exist as two enantiomers, the D form and the L-form.

The protein may be manufactured synthetically (by means of organic synthesis in a cell-free system that makes use of a ribosome system that may or may not be artificial) or it may be manufactured naturally by cells that may or may not be genetically manipulated, or by a single cell organism (e.g. by a bacterium or a yeast cell) or a multicellular organism.

The protein could be modified in many ways. Typical modifications are post-translational modifications that include lipids, acetate groups, phosphate groups, or carbohydrates. The protein could also chemically modified with e.g. biotin groups.

The protein can be a multimer consisting of identical subunits (homomer) or different proteins (heteromer) which are hold together by covalent bonds or non-covalent interactions.

The term "binding affinity" used herein refers to the strength of the affinity between the CD44ex9 binding protein and CD44v5 domain and is described by the dissociation constant K_{D}. The K_{D} value for the binding affinity between the CD44ex9 binding protein and CD44v5 domain may be determined by equilibrium methods, (e.g. enzyme-linked immunoabsorbent assay (ELISA) or radioimmunoassay (RIA)) or kinetics (e.g. BIACORE™ analysis), for example.

"K_{D}" refers to the relative binding affinity between the CD44ex9 binding protein and CD44v5 domain. High K_{D} values represent low binding affinity.

In the context of the invention the term "acute treatment" refers to a short-term medical treatment, usually in a hospital, for patients having an acute illness or injury or recovering from surgery. The term "post-acute treatment" refers to a mid-term medical treatment, usually in a hospital, for patients having an acute illness or injury or recovering from surgery.

The term "treating" or "treatment" refers to any medical measure for preventing, reducing, mitigating or curing physiological disorders within a mammal, in particular a human, in the need thereof. According the invention the treatment also encompasses a targeted drug delivery.

A "therapeutically effect amount" is defined as the amount of active ingredient that will reduce the symptoms associated with a neurological or neurodegenerative disease, such as stroke. "Therapeutically effective" also refers to any improvement in disorder severity or the frequency of incidences compared to no treatment. The term "treatment" encompasses either curing or healing as well as mitigation, remission or prevention.

The term "expression vector" refers to vectors that have the ability to incorporate and express heterologous DNA fragments in a foreign cell. Many prokaryotic and eukaryotic expression vectors are known and/or commercially available. Selection of appropriate expression vectors is within the knowledge of the skilled person.

As disclosed herein the term "nanoparticle" refers to a particle with a size below 1 µm and preferably between 1 and 100 nm. Thus, the term "nanoparticle" includes nanocrystals made from semiconductor materials (so called quantum dots), as well as nanoparticles consisting of a Noble metal cluster, rare-earth based inorganic luminescent nanoparticles, superparamagnetic iron oxide nanoparticles (IONPs), block-copolymer micelles, nanocells, dendrimers, nanotubes, polymersomes, XPclad® nanoparticles, and nanoparticles consisting of amorphous silica surrounded by a crystalline luminescent calcium phosphate layer (e.g. ORMOBEAD ®).

### Literature

Dembski S, Probst J, Kockenbring T and Barth S, News Analytik, 18.1.2013; p. 1 -3.
Dembski S, Rupp S, Milde M, Gellermann C, Dyrba M, Schweizer S, Batentschuk M, Osvet A, Winnacker A, Optical Materials, 2011, p.1106 -1110. (2011a)
Dembski S, Gellermann C, Probst J, Klockenbring T, Barth S, GIT-Labor-Fachzeitschrift 01/2011: 48-49. (2011b)
Deonarain MP, Kousparou CA, Epenetos AA, MAbs, 2009, 1(1): 12-25.
Goding JW, Monoclonal Antibodies: Principles & Practice (Academic Press, London, 2nd edition, 1986), p. 61-63.
Günthert U, Hofmann M, Rudy W, Reber S, Zöller M, L Haubmann L, Manzku S, Wenzel A, Ponta A, Herrlich P: "A new variant of glycoprotein CD44 confers metastatic potential to rat carcinoma cells", Cell 1991, 65: 13-24.
Hale LP and Haynes BF, J Immunol. 1992, 149(12): 3809-3816.
Jalkanen ST, Bargatze RF, Herron LR, Butcher EC. A lymphoid cell surface glycoprotein involved in endothelial cell recognition and lymphocyte homing in man. Eur J Immunol. 1986 Oct;16(10):1195-1202.
Johnson P & Ruffell B, Inflamm Allergy Drug Targets, 2009, 8(3): 208 - 220.
Kampmeier F, Ribbert M, Nachreiner T, Dembski S, Beaufils F, Brecht A and Brecht F, Bioconjugate Chem 2009, 20(5): 1010 - 1015.
La Fleur L, Johansson A-C, Roberg K, PLoS ONE 2012, 7(9): e44071
Nagano O and Saya H, Cancer Sci 2004, 95(12): 930-935.
Piotrowicz RS, Damaj BB, Hachicha M, Incardona F, Howell SB and Finlayson M, Mol Cancer Ther 2011, 10(11): 2072 - 2082.
Ponta H, Sherman L, Herrlich PA, Nat Rev Mol Cell Biol. 2003, 4(1): 33-45.
Probst J, Dembski S, Milde M, Rupp S, Expert Review of Molecular Diagnostics 2012, 12(1): 49-64.
Seiter BS, Arch R, Reber S, Komitowski D, Hofmann M, Ponta H, Herrlich P, Matzku S, and Zoller M. Prevention of tumor metastasis formation by anti-variant CD44. J Exp Med, 177: 443-455, 1993.
Saturag et al 2000; British Journal of Nutrition; 2000, (84), 791-802
Singh R and Lillard Jr. JW, Exp Mol Pathol, 2009, 86(3): 215-223.
Tempfer C, Lösch A, Heinzl H, Häusler G, Hanzal E, Kölbl H, Breitenecker G, Kainz C, Eur J Cancer, 1996, 32A: 2023-2025.
Vizoso FJ, Fernandez JC, Corte MD, Bongera M, Gava R, Allende MT, Garcia-Muniz JL, Garcia-Moran M, J Cancer Res Clin Oncol, 2004, 130: 679-686.
Xie S, Lee S, Chen X, Adv Drug Deliv Rev 2010, 62(11): 1064-1079.
Zhang S, Wu CC, Fecteau JF, Cui B, Chen L, Zhang L, Wu R, Rassenti L, Lao F, Weigand S, Kipps TJ, Proc Natl Acad Sci USA, 2013, 110: 6127-6132.

### Figure legends

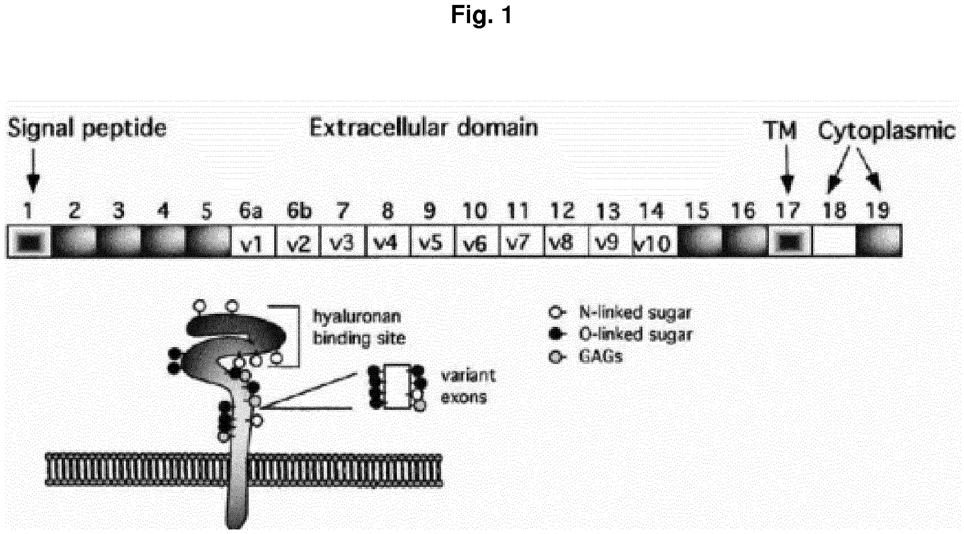

- Fig. 1:: Genomic structure and protein domains of CD44.
- Fig. 2:: (A) Schematic outline of the strategy of the yeast-two-hybrid screening. (B) Vector used for the Y2H-screen.
- Fig. 3:: Amino acid sequences of the positive CD44-interacting clones together with the consensus sequence derived from it.
- Fig. 4:: Labeling of HT29 cells with the nanoparticle conjugate Q_CA19-9-3.
- Fig. 5:: Negative control for labeling of HT29 cells with the nanoparticle conjugate Q_CA19-9-5.
- Fig. 6:: Quantification of HT29 cell-labeling with the nanoparticle conjugate Q_CA19-9-3 or the negative control MBP-nanoparticle.
- Fig. 7:: Competition of HT29 cell-labeling with the nanoparticle conjugate Q_CA19-9-3 by addition of increasing amounts of the free target CA19-9.
- Fig. 8:: Labeling of Colo29 cells with the nanoparticle conjugate Q_CA19-9-3.
- Fig. 9:: Negative control for labeling of Colo29 cells with the nanoparticle conjugate Q_CA19-9-5.
- Fig. 10:: Labeling of SW116 cells with the nanoparticle conjugate Q_CA19-9-3.
- Fig. 11:: Negative control for labeling of SW116 cells with the nanoparticle conjugate Q_CA19-9-5.
- Fig. 12:: Labelling of mucinous adenocarcinoma tissue (sample No. 35) with a bispecific nanoparticle Q_CEA/CA19-9_12 conjugated to an anti-CEA and an anti-CA-19-9 antibody. In the four quadrants, the following conditions were analysed:
Upper left: Labeling with Q_CEA/CA19-9_12
Upper right: Labeling with CEA/CA19-9_12 by addition of the free target MBP-CEA (25 µM)
Lower left: Labeling with Q_CEA/CA19-9_12 by addition of the free target Sialyl Lewisₐ (250 µM)
Lower right: Labeling with Q_CEA/CA19-9_12 by addition of the free targets MBP-CEA (25 µM) and Sialyl Lewisₐ (250 µM)

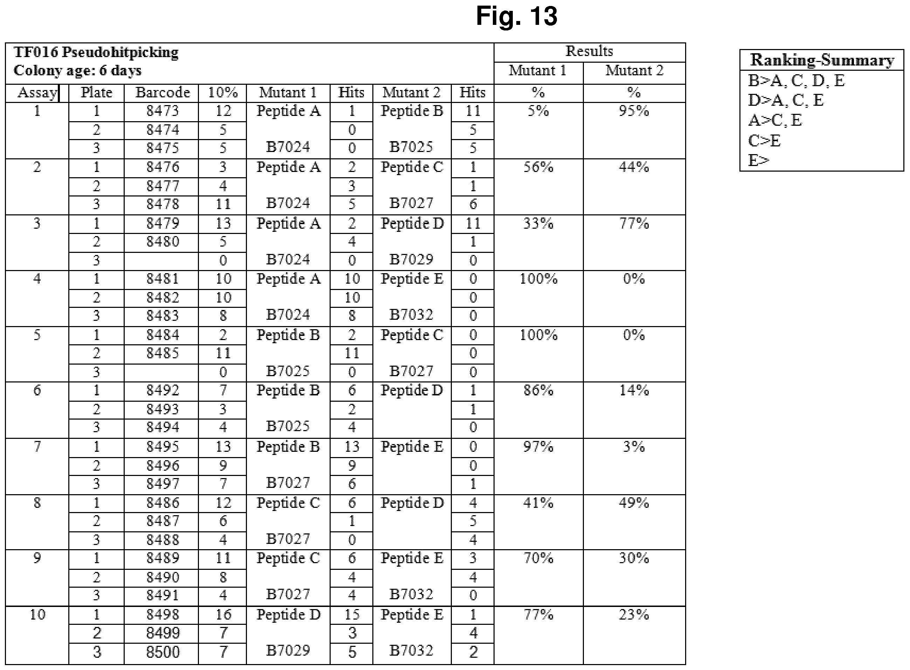

- Fig. 13:: Affinity ranking experiments for peptides A to E: Results of the Pseudohitpicking assay I

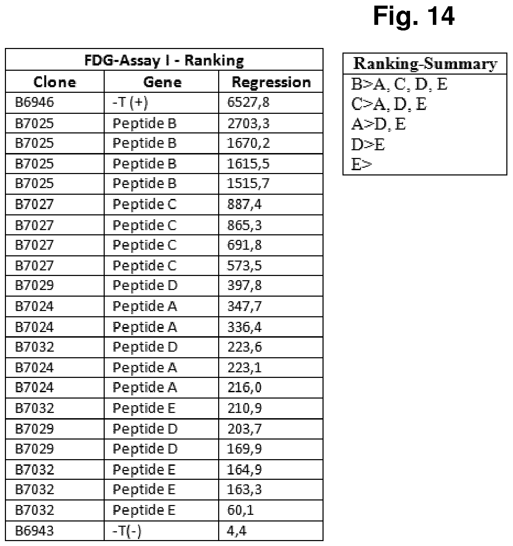

- Fig. 14:: Affinity ranking experiments for peptides A to E: Results of the FDG assay I

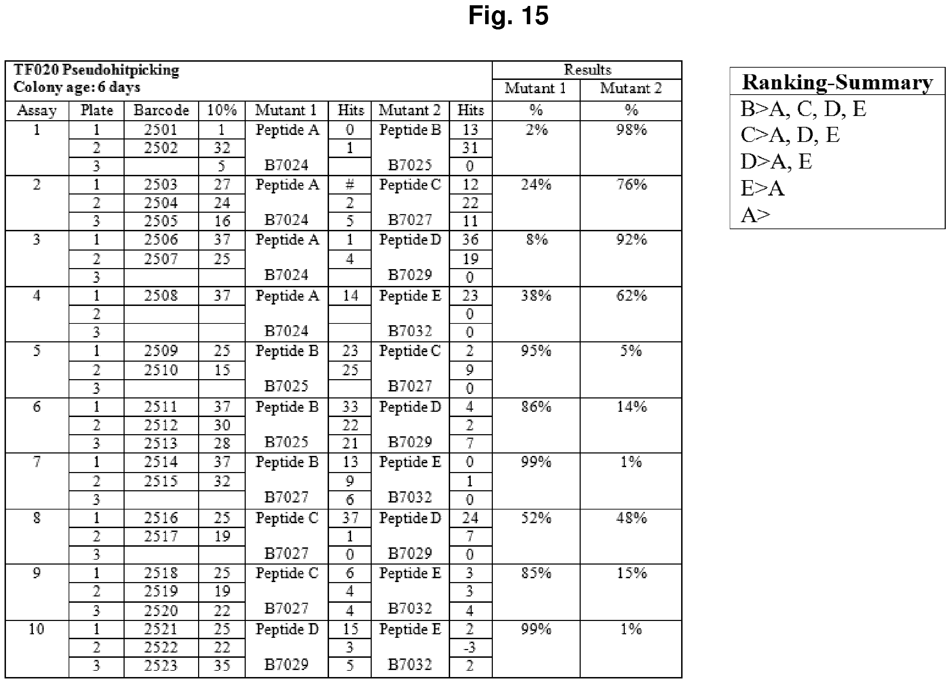

- Fig. 15:: Affinity ranking experiments for peptides A to E: Results of the Pseudohitpicking assay II

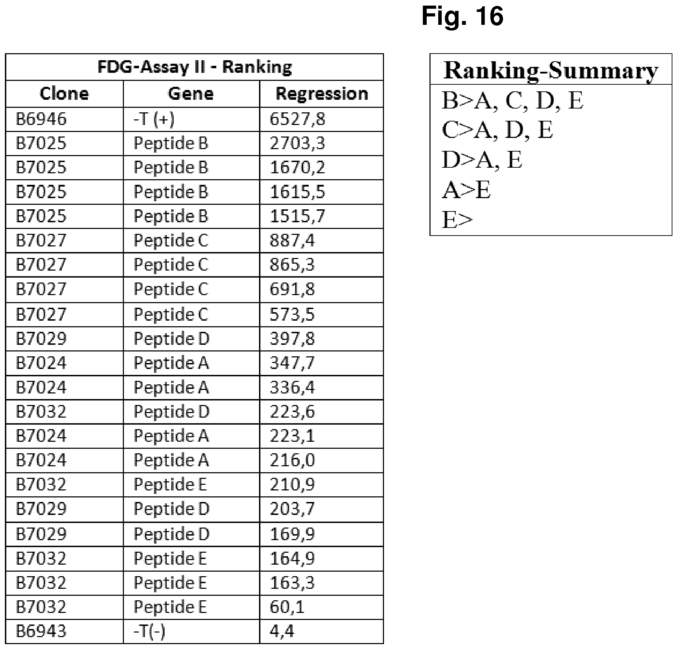

- Fig. 16:: Affinity ranking experiments for peptides A to E: Results of the FDG assay II

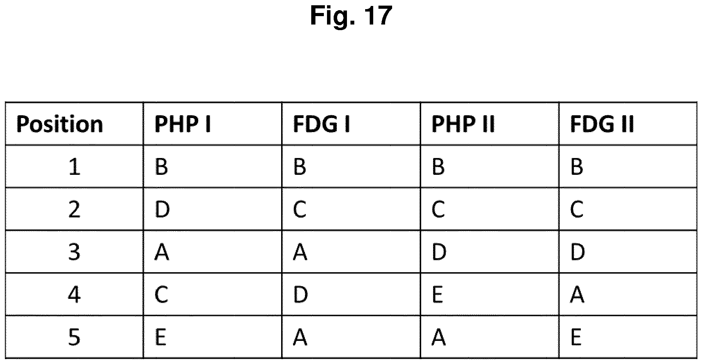

- Fig. 17:: Overall summary of the Affinity ranking experiments for peptides A to E

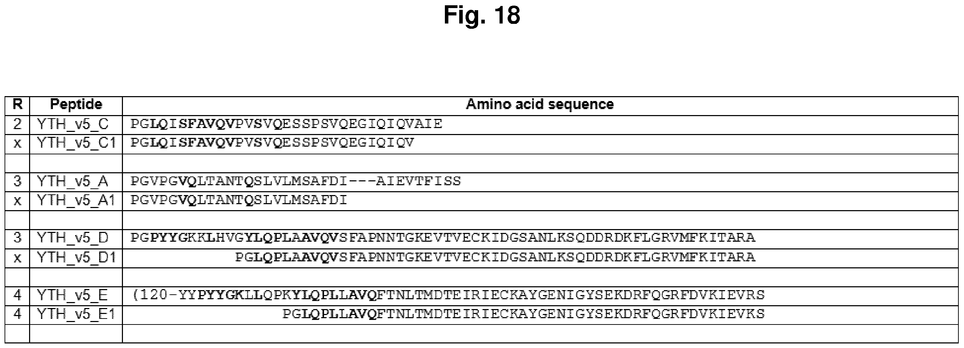

- Fig. 18:: Diagram showing the deletion mutants for the peptides A, C, D and E

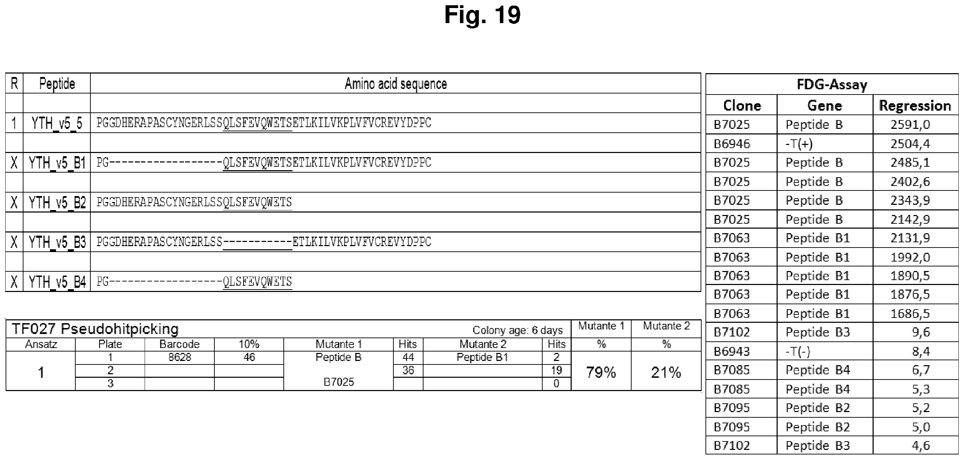

- Fig. 19:: Diagram showing the deletion mutants for the peptide B (left side) and the results of their affinity analysis in the FDG assay (right side)

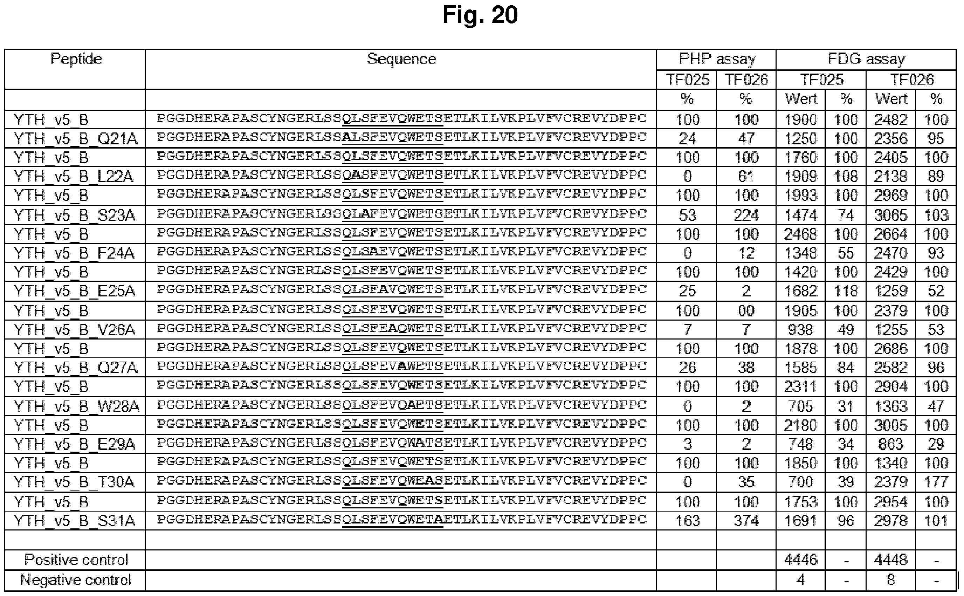

- Fig. 20:: Table showing the alanine scanning mutants for the peptide B and the results of their affinity analysis in PHP and FDG assays, both performed in twice.

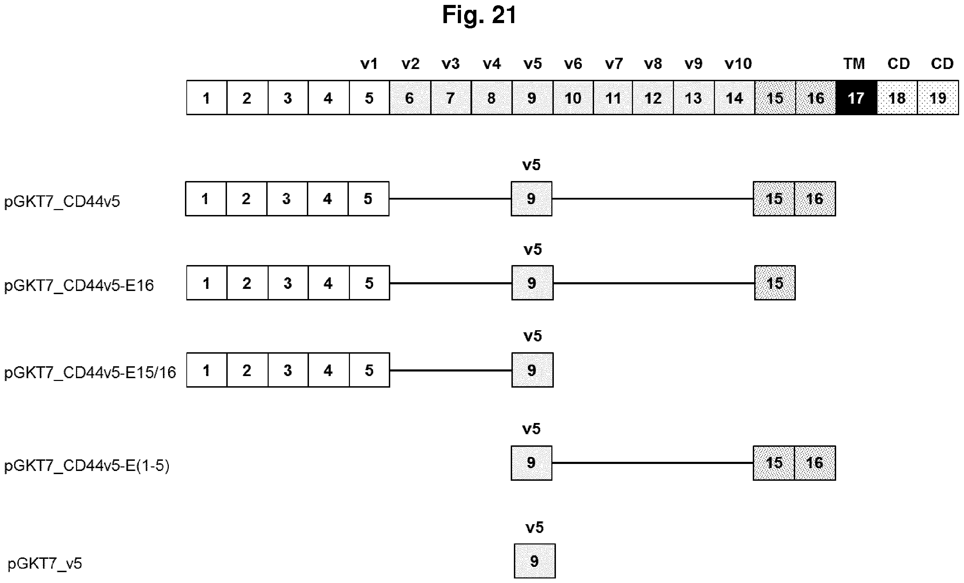

- Fig. 21:: Diagram showing the alternative CD44 constructs that were generated for the binding analyses.

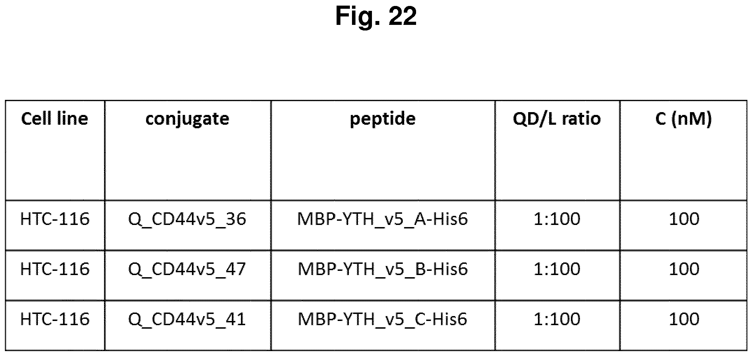

- Fig. 22:: Diagram giving an overview on the *in vitro* cytochemical experiments performed with CD44v5-binding peptides A, B or C coupled to fluorescent nanoparticles.

### EXAMPLES

### Example 1: Isolation of CD44ex9-bindingproteins

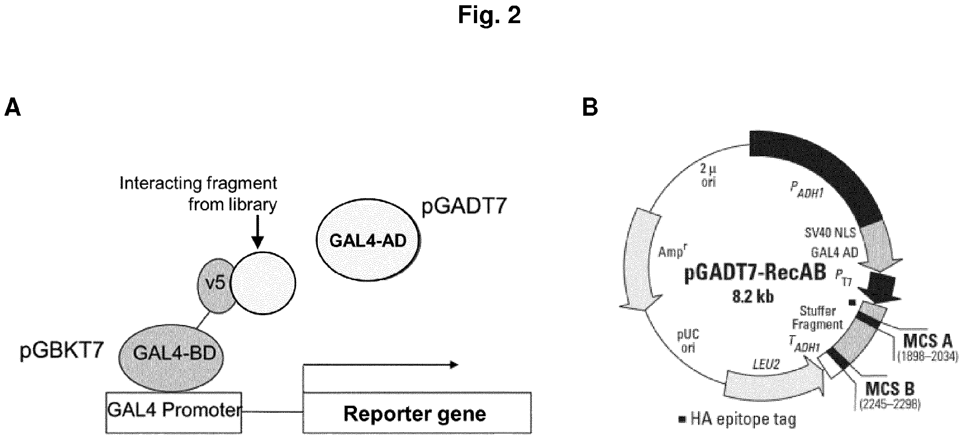

For the isolation of proteins or protein fragments binding to the v5 domain of CD44 a competitive two hybrid screening was performed according to the method disclosed in EP 1 721 974 A1. As bait, the v5-fragment was fused the GAL4 binding domain. The respective ORF was cloned into a plasmid vector and designated as pGBKT7 (see Figure 2). A human cDNA library with fragments fused to the GAL4-activation domain was used as a prey (vector pGADT7-RecAB; see Figure 2).

The screening was performed under stringent selection conditions and resulted in the identification of 39 positive clones. After selective co-transformation of the bait and the pray plasmid, 21 of the 39 clones remained positive. The 21 clones were sequenced and analysed by BLAST search. As result the 21 sequences could be reduced to 12 different sequences since four sequences were identified twofold and one sequence was present in four clones.

Furthermore, a sequence comparison revealed that these five sequence families show extensive sequence homologies allowing the formation of a consensus sequence (see Figure 3).

### Example 2: In vitro detection of antigen-expressing tumor cells

### 2.1 Background and Objective

In order to demonstrate the ability of the protein-quantum dots conjugates to specifically detect antigen-expressing tumor cells, a tumor specimen or in vitro cultivated tumor cells were analysed by fluorescence and in parallel by immunohistochemistry for expression of the respective antigen. As a first antigen, the carcinoembryonic antigen (CEA) was analysed. CEA is a glycoprotein involved in cell adhesion. As a detection-ligand, the anti CEA antibody was conjugated to the quantum dot QDBP-655 (charge #773780). Furthermore, the cancer antigen 19-9 (CA19-9) was analysed. CA19-9 is a sialylated Lewis (a) antigen and represents a tumor marker that is used primarily in the management of pancreatic cancer.

### 2.2. Samples

### 2.2.1 Cell culture samples

In a first set of experiments, the in vitro cultivated human cancer cell lines HT29, Colo25 and SW116 were used. HT29 is an adherent colorectal adenocarcinoma cell line, Colo25 a human colon carcinoma cell line and SW116 a colorectal cancer cell line.

### 2.2.2. Tumor sample

In a separate set of experiments, a sample taken from a human tumor was used. The tumor sample No. 35 was resected in 2011 from coecum and was diagnosed as a mucinous adenocarcinoma. It was classified according the TNM classification as pT4No(0/18)G3Ro. This sample which was not otherwise pretreated was prepared and fixed as follows.

### Preparation and fixation of tissue sections

The resected tumor sample, stored at -70°C was used to prepare cryosections with a thickness of 5 µm using a HM560 MV cryostate (Thermo Scientific, Walldorf, Germany). The sections were dried for 60 to 120 min at room temperature and incubated with cold acetone (-20°C) for 10 minutes. After a further drying step for 10 min at room temperature, the sections were stored over night at -70°C.

The sections were defrosted in a closed box for 30 min at room temperature. Fixation was performed by incubation in a solution containing 20 mg/ml dimethyl suberimidate (DMS) and 20 mM CaCl₂ in 250 mM Tris-HCI buffer pH 8.0. Afterwards the sections were washed for 5 minutes in D-PBS-T buffer containing 130 mM NaCl, 7 mM Na₂HPO₄, 3 mM KH₂PO₄ and 0.1% Tween 20 by a quenching step for 20 minutes in a buffer containing 130 mM NaCl, 7 mM Na₂HPO₄, 3 mM KH₂PO₄ and 200 mM glycerine. In a final step the sections were washed for 5 minutes in D-PBS-T buffer.

### Counterstaining of the tissue sections.

Counterstaining was performed using the Dako Autostainer Plus (DAKO Deutschland GmbH, Hamburg, Germany) according the following protocol:
- Rinsing with D-PBS-T buffer
- Blocking for 60 min with 1% BSA / 5% goat serum in D-PBS buffer
- Blowing off
- Incubation with 2 x 100 µl conjugate (20nM in 1% BSA / 5% goat serum in D-PBS buffer) for 60 min
- Rinsing for three times with D-PBS-T
- Blowing off
- Incubation with 2 x 100 µl primary antibody (20nM in 1% BSA / 5% goat serum in D-PBS buffer) for 60 min
- Rinsing for three times with D-PBS-T
- Blowing off
- Incubation with 2 x 100 µl secondary antibody (20nM in 1% BSA / 5% goat serum in D-PBS buffer) for 60 min
- Rinsing for three times with D-PBS-T
- Blowing off
- Incubation with 3 x 200 µl Hoechst 33342 (2µg/ml in D-PBS buffer) for 10 min
- Rinsing for three times with D-PBS-T
- Embedding in Mowiol/Triethylenediamine (DABCO)

### Microscopic Evaluation

The sections as prepared above were evaluated with a reverse microscope Axiovert 200 with Axiocam HrM and a Axiocam Hrc CCD camera system, HXP 120 C illuminating device. The pictures were analysed using the Axiovision software (version 4.8; Carl Zeiss, Oberkochen, Germany).

### 2.3 Protein-quantum dot conjugates

For antigen detection the quantum dots as listed in the following table were prepared:

| Conjugate | Type of QDBP-655 quantum dot | Ligand | QDBP/Ligand ratio |
|---|---|---|---|
| Q561 | #773780 | SI6166 | N/A |
| Q_CA19-9-20 | #773780 | MBP-NS-19-9-scFv-His₆ + SI6166 | 1:50 |
| Q_CEA/CA19-9_9 | Q561 | MBP-NS-19-9-scFv-His₆ + SI6166 | 1:10 |
| Q_CEA/CA19-9_10 | Q561 | MBP-NS-19-9-scFv-His₆ + SI6166 | 1:20 |
| Q_CEA/CA19-9_11 | Q561 | MBP-NS-19-9-scFv-His₆ + SI6166 | 1:30 |
| Q_CEA/CA19-9_12 | Q561 | MBP-NS-19-9-scFv-His₆ + SI6166 | 1:40 |
| Q_CEA/CA19-9_13 | Q561 | MBP-NS-19-9-scFv-His₆ + SI6166 | 1:50 |
| Q_CEA/CA19-9_1 | Q561 | MBP-NS-19-9-scFv-His₆ + SI6166 | 1:50 |

The conjugate Q561 is a conjugate between the quantum dot QDBP-655 (batch #773780) and the ligand SI6166. QDBP-655 has a hydrodynamic diameter of 6 nm and possesses a Ni-NTA ligand. The ligand SI6166 is an CEA-binding protein having a hydrodynamic diameter of 2.1 nm, and the final conjugate exhibits a hydrodynamic diameter of 10.1 nm. The anti CEA protein is coupled to the quantum dots.

The quantum dots Q_CA19-9-x are conjugates between the quantum dot QDBP-655 (batch #773780) and the anti-CA 19-9 antibody "MBP-NS-19-9-scFv-His6". This anti-CA19-9 antibody which is a recombinant antibody mimetic consisting of the variable region of the heavy chain (VH) and the light chain (VL) and can be described as a "single chain fragment of variable regions", scFv. This scFv fragment is fused to a His-tag which is used for the coupling to the quantum dot. The anti-CA 19-9 antibody "MBP-NS-19-9-scFv-His6" is also denominated as SI6950.

The quantum dots Q_CEA/CA19-9_x are bispecific conjugates, whereby the quantum dot Q561 harboring the anti CEA-antibody SI6166 is further conjugated with the anti-CA 19-9 antibody SI6950.

Different batches of the conjugate Q_CEA/CA19-9_x were prepared differing by the ratio of the quantum dot to the ligand (see last column of the table).

### 3. Results of CA19-9 conjugated nanoparticles

### 3.1 HT29 cells

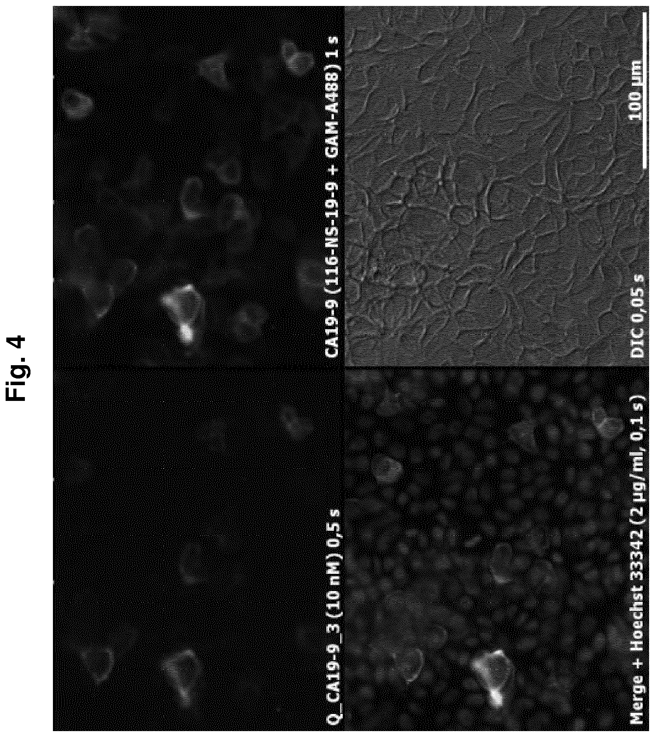

As shown in upper left panel of Fig. 4, the conjugate Q_CA19-9-3 labels the cell membrane of the HT29 cells. A staining of the cells with the anti-CA19-9 antibody detected by Alexa Fluor 488 fluorescence (upper right panel) shows a perfect co-localization of the signals showing the specificity of the conjugate binding. In the lower right panel the cells are shown by differential interference contrast (DIC) microscopy.

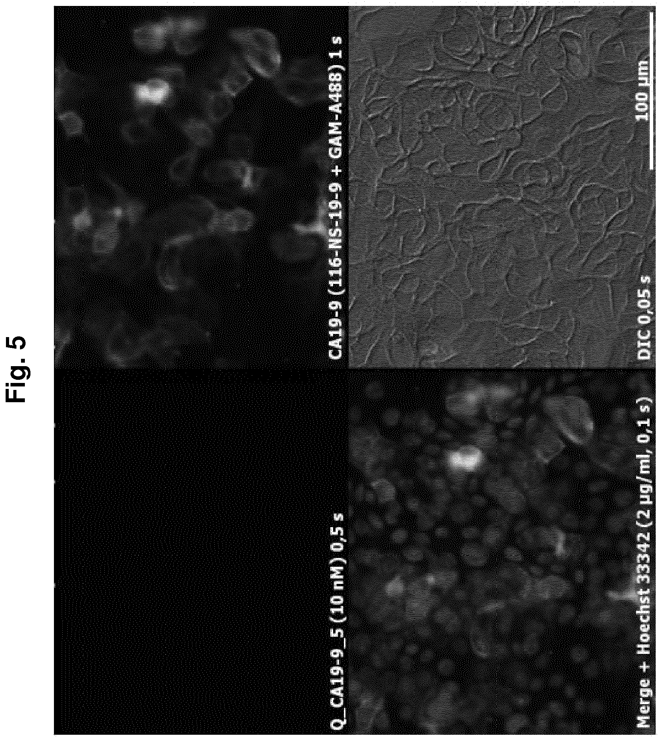

In the negative control a quantum dot conjugated to MBP (MBP-His/QDP655) was used. As shown in the upper left panel of Fig. 5, no labeling could be detected. The presence of the CA19-9 antigen was verified by staining of the cells with the anti-CA19-9 antibody detected by Alexa Fluor 488 fluorescence (upper right panel).

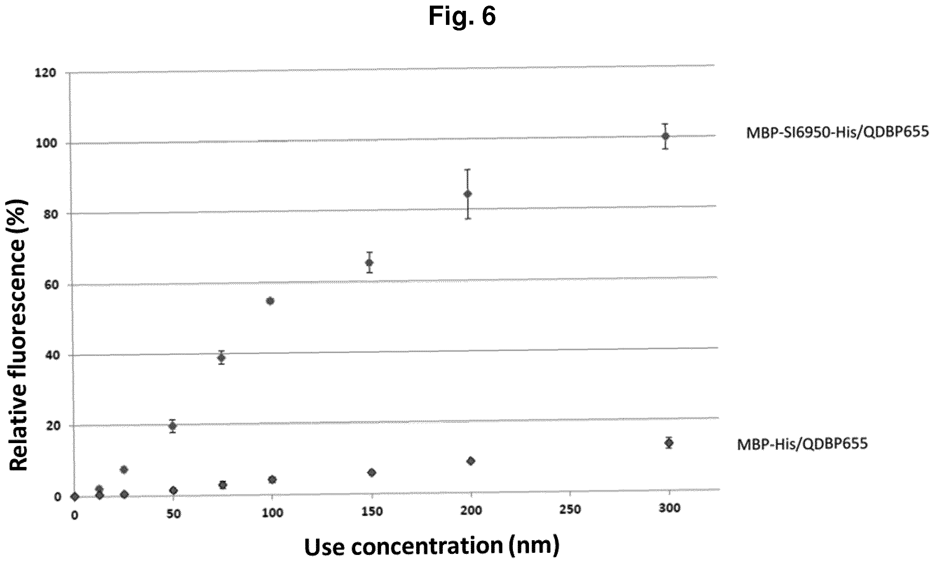

The specific labeling of the CA19-9 antigen was also shown by a titration experiment. A shown in Fig. 6, an increasing amount of the conjugate Q_CA19-9_3 leads to an increased labeling of the HT29 cells as demonstrated by increasing relative fluorescence. In contrast, the negative control MBP-His/QDP655 leads only to a sparse fluorescent labeling which show no major increase at higher conjugate concentrations.

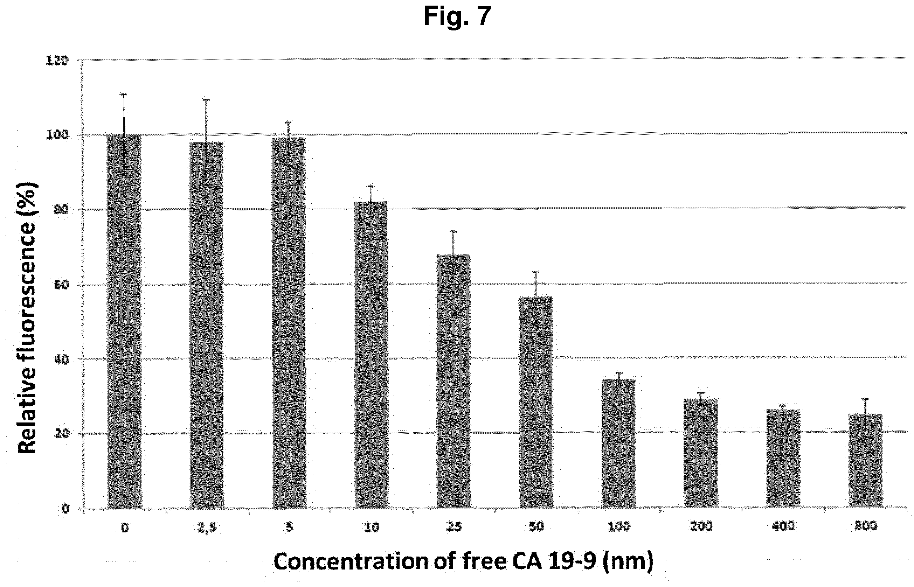

The specificity of the CA19-9 labeling was further verified by a competition experiment as shown in Figure 7: An increasing amount of free CA19-9 antigen added to the labeling mixture leads to a decreased CA19-9 labeling.

### 3.2 Colo29 cells

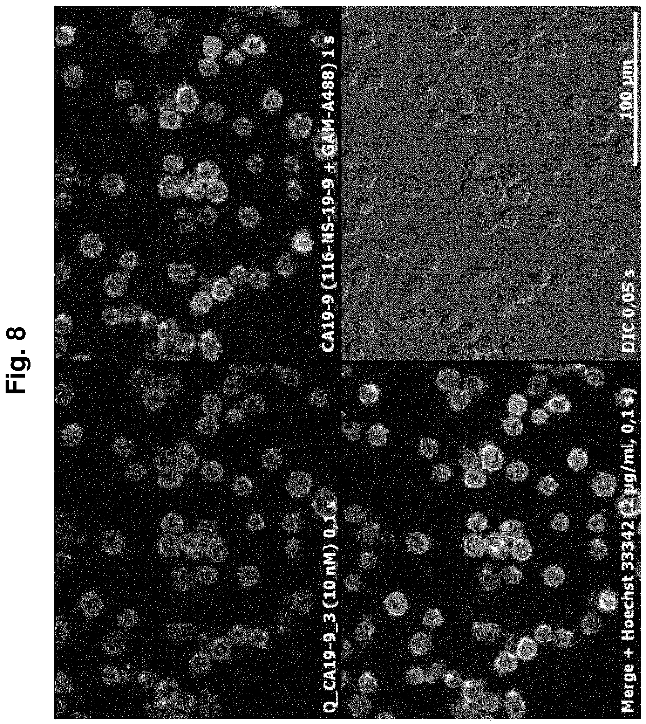

As shown in upper left panel of Fig. 8, the conjugate Q_CA19-9-3 labels the cell membrane of the Colo29 cells. A staining of the cells with the anti-CA19-9 antibody detected by Alexa Fluor 488 fluorescence (upper right panel) shows a perfect colocalization of the signals showing the specificity of the conjugate binding. In the lower right panel the cells are shown by differential interference contrast (DIC) microscopy.

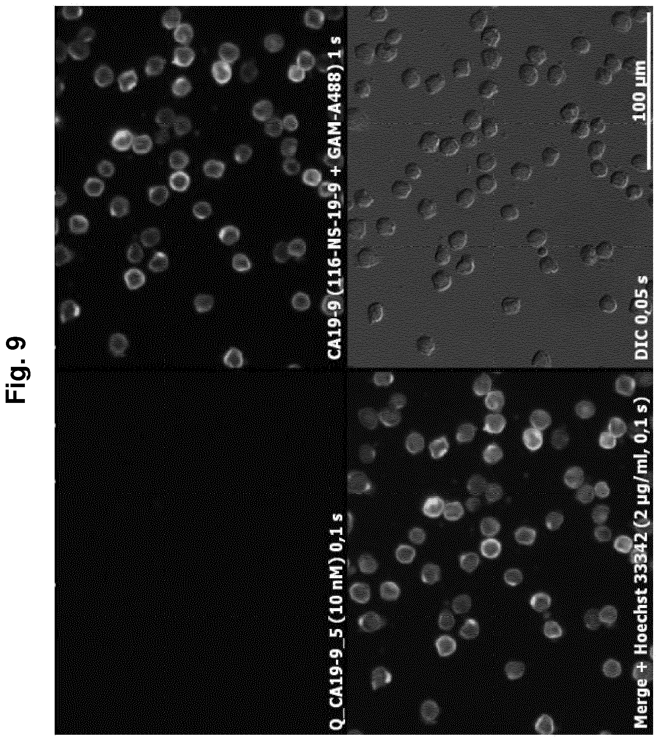

In the negative control a quantum dot conjugated to MBP (MBP-His/QDP655) was used. As shown in the upper left panel of Fig. 9, no labeling could be detected. The presence of the CA19-9 antigen was verified by staining of the cells with the anti-CA19-9 antibody detected by Alexa Fluor 488 fluorescence (upper right panel).

### 3.3 SW116 cells

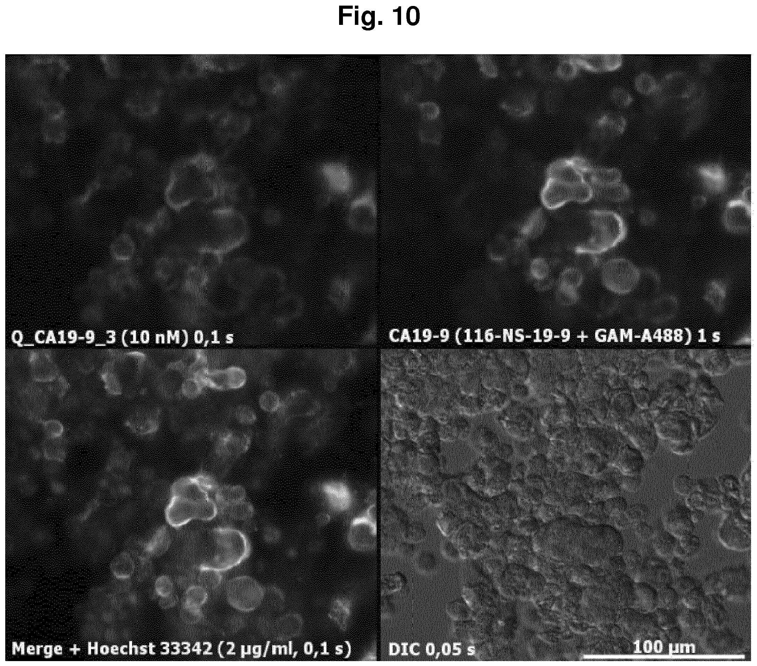

As shown in upper left panel of Fig. 10, the conjugate Q_CA19-9-3 labels the cell membrane of the Colo29 cells. A staining of the cells with the anti-CA19-9 antibody detected by Alexa Fluor 488 fluorescence (upper right panel) shows a perfect colocalization of the signals showing the specificity of the conjugate binding. In the lower right panel the cells are shown by differential interference contrast (DIC) microscopy.

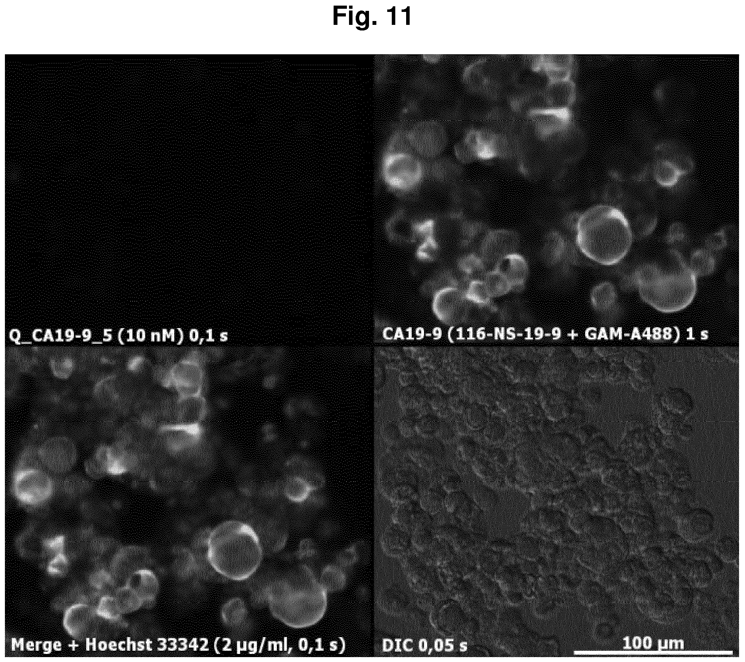

In the negative control a quantum dot conjugated to MBP (MBP-His/QDP655) was used. As shown in the upper left panel of Fig.11, no labeling could be detected. The presence of the CA19-9 antigen was verified by staining of the cells with the anti-CA19-9 antibody detected by Alexa Fluor 488 fluorescence (upper right panel).

### 4. Results of bispecific conjugated nanoparticles

This experiment was performed in order to test the ability of the bispecific conjugated nanoparticle to interact with both antigens in a given target. For this purpose the nanoparticle conjugated with both the anti-CA19-9 antibody and the anti CEA antibody was used to detect the respective antigens on the tumor cells of above described sample No. 35. The specificity was demonstrated by inhibition with the free targets MBP-CEA and 19-9.

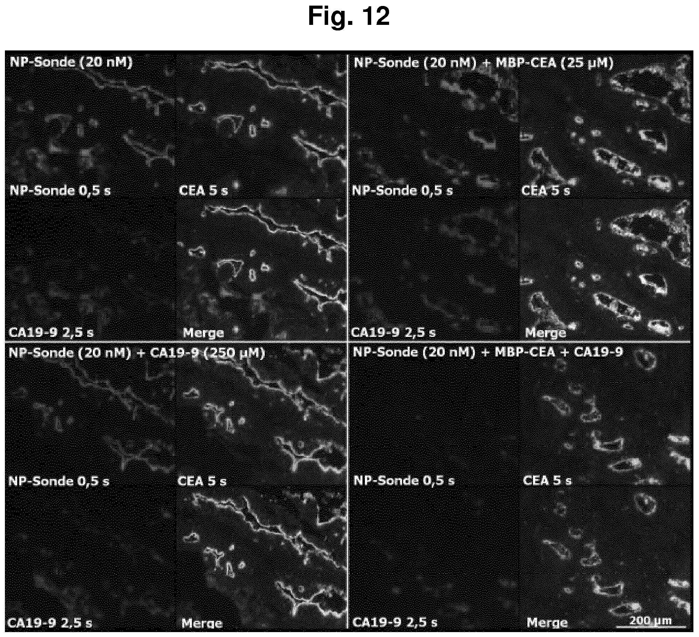

As shown in the upper left part of Figure 12, the bispecific nanoparticles labeled cell membranes of the tumor tissue. A staining of the tumor tissue with an anti-CA19-9 antibody or an anti CEA-antibody showed in each case a partial colocolization with combines to the complete labeling profile of the nanoparticle,

When the bispecific nanoparticle is used in combination with the free target MBP-CEA, a labeling can be observed that shows a perfect colocalization with the staining of the anti-CA-19-9 antibody (see Figure 12, upper right quadrant). Hence, the free target inhibits the interaction with the cellular-bound CEA but still allows a labeling of the CA19-9.

As shown in the lower left quadrant of Figure 12, the addition of the free target Sialyl-Lewisₐ inhibits the interaction with the cellular CA19-9 antigen but retains the CEA-labeling.

In a final experiment the bispecific nanoparticles were combined with both free targets, the MBP-CEA and Sialyl-Lewisₐ (see lower right quadrant of Figure 12). As a result the labeling was completely inhibited (upper left corner of the quadrant). The colocalized expression of both antigens was verified by staining with the respective anti-CEA and CA19-9 antibodies.

### Example 3: Characterization of CD44ex9-binding proteins by affinity ranking

### Introduction:

The CD44ex9-binding proteins as identified in the competitive two hybrid screening of Example 1 were further analysed by two different affinity assays to establish an affinity ranking.

### Methods:

As a first assay a pseudohitpicking assay was used, which was performed as follows: In order to compare the affinity of two different peptides, colonies expressing the first peptide or the second peptide, respectively, were plated on the first or second half of an agar plate. The threshold value for defining a colony as a "hit" was then determined, that a percentage of colonies of the first peptide, which is defined in advance, in the reference region of the plate are picked as "hits". In the same experiment, the colonies of the second peptide are scanned and picked. The number of the colonies of the second peptide are then compared to the number of colonies of the first peptide and expressed as a percentage. Based on this percentage the relative interaction strength (i.e. the affinity to the CD44 peptide) can be derived.

The FDG assay is based on the reporter system of the two hybrid scanning system. Hereby the GAL4-binding domain with the CD44v5 peptide interacts with the respective peptides fused to the GAL4-actvation domain. After complex formation two independent reporter genes are activated, the first one encodes a fluorescent protein, the second reporter gene encodes the enzyme beta-galactosidase, whose enzymatic activity can be determined by using the fluorogenic substrate Fluorescein di-beta-D-galactopyranoside (FDG).

In the pseudohitpicking assay both reporter systems will be considered: At first the fluorescence of endogen reporter gene and after incubation with a fluorescence substrate the activity of the second enzyme-encoding reporter gene. Therewith, the results of two different reporter genes can be determined within one experiment.

As shown in Figures 13 to 16, ten different combinations of two of the five peptides of SEQ ID No. 1 to 5 were compared in the pseudohitpicking assay and in the FDG assay, performed in parallel. Hereby, two independent experiments were conducted as shown in Figures 13 to 16.

### Results:

Both experiments showed that peptide B (SEQ ID No. 3) has the highest affinity towards the CD44v5 peptide followed by peptide C and peptide D. The overall summary of the affinity ranking analysis is given in Figure 17.

### Example 4: Characterization of CD44ex9-binding proteins by deletion

### Introduction:

For the five peptides a deletion-based epitope mapping was performed in order to identify and characterize the binding sites of these peptides. The results represent an important information which can aid in the development of CD44v5-associated therapeutics and diagnostics.

### Methods:

For all five peptides A to E, deletion mutants were generated and tested in the PHP and in the FDG assay. For the peptides A, C, D and E an N- or C-terminal deleted fragment was generated and denominated as peptide A1, C1, D1 and E1. The sequences of the deletion mutants for peptides A, C; D and E are depicted in Figure 18.

For the peptide B, altogether four deletion mutants, namely the peptides B1 to B4 were generated as shown in Figure 19.

The peptides were comparatively tested in the PHP and the FDG assay.

### Results:

For the peptide B, the N-terminal deletion of 18 amino acids (peptide B1) resulted in a peptide that slightly decreased activity (see Figure 19, right side). Hence, this N-terminal region is not crucial for the interaction to the CD44v5 peptide and therefore peptide B1 represents a core sequence for the interaction with CD44v5.

As shown in Figure 19, peptides with N- and C-terminal deletion (peptide B4), with C-terminal deletion only (peptide B2) and with deletion of the core sequence "QLSFEVQWETS" (peptide B3) do completely loss to ability to bind to CD44v5. This result is in perfect agreement with the profile of peptide B1 showing that at least the core sequence together with the C-terminal part are required for CD44v5 binding.

The peptides A1 and C1 differ from their parent peptides A and C by the lack of the C-terminal 3 or 8 amino acid long sequence including the motif "AIE" of the consensus sequence. In both cases the binding to CD44v5 is almost completely abolished showing the relevance of this part of the consensus sequence.

The peptides D1 and E1 differ from their parent peptides D and E by the lack of a N-terminal sequence including the motif "PYYGKXLXX" of the consensus sequence. In both cases the binding to CD44v5 is not diminished. Whereas peptide D1 is of similar affinity, the peptide E1 show even a stronger binding towards CD44v5 compared to the parent peptide E.

These results are in perfect accordance with the results for the peptide B as described above. The N-terminal region is not necessary for CD44v5 binding and a shortened peptide and its respective consensus sequence as a core sequence represents the preferred peptide motif for CD44v5-associated diagnosis and therapy.

### Example 5: Characterization of CD44ex9-binding proteins by Alanine scanning

### Introduction:

For the most affine peptide B an alanine scan of the core sequence was performed in order to identify those amino acids which are crucial for CD44v5 binding. As shown by the mutated peptide B3, the presence of the core sequence "QLSFEVQWETS" is mandatory for CD44v5 binding.

### Methods:

The peptide B was used to generate altogether 11 mutants, whereby every amino acid of the core sequence "QLSFEVQWETS" was independently substituted by the amino acid alanine (see Figure 20). These mutants were tested in comparison with the peptide B in the PHP and in the FDG assay. For all five peptides A to D, deletion mutants were generated and tested in the PHP and in the FDG assay.

### Results:

As shown in Figure 20, the substitution of the first 10 amino acids of the core sequence does not led to a significant change in affinity towards CD44v5. However, the exchange of the 11th amino acid serine of the underlined core sequence by alanine resulted in a peptide with higher affinity.

Hence, there is no single amino acid within the core sequence, that is crucial for CD44v5 binding but it has to be assumed that these amino acids bind in a coordinative manner to the target.

However, based on the results an even improved peptide B and an improved consensus sequence with a Ser-Ala exchange in the 11^{th} amino acid were identified (see consensus sequences of SEQ ID No. 58 to 62).

### Example 5: Binding analysis of CD44ex9-bindinqproteins towards different CD44 variants.

As shown in Figure 21, altogether four different CD44 variants were generated that contain the v5 domain in combination with different domains. As revealed by PHP and FDG assays, the CD44ex9-binding proteins of the invention were able to bind also these CD44 variants. As a negative control the CD44 variant pGKT7_CD44 was used encoding only the domains 1 to 5 and 15, 16 (not shown here). As expected, the CD44ex9-binding proteins do not show any binding to this CD44 protein.

### Example 5: Binding analysis of nanoparticles coupled to CD44ex9-binding proteins towards in vitro cultivated CD44v5-expressing tumor cells

In order to underscore the diagnostic and therapeutic potency of the CD44ex9-binding proteins, the peptides A, B and C were expressed as MBP-fusion proteins containing a His-tag, coupled to quantum dots and used for immunocytochemical analysis of CD44-expressing tumour cells.

### Methods:

The peptides were expressed as MBP-(YTH_v5_A/B/C)-His₆ fusion proteins in E.coli, purified using Ni-NTA affinity chromatography and coupled via the HiS₆₋Tag to the QDBP-655 quantum dots. These quantum dots possess a CdSe core, a ZnS shell and a passivation layer comprising an imidazole compound.

The characteristics of the QDBP-655 nanoparticles as herein can be summarized as follows:
The nanoparticles have a cadmium selenide [CdSe] core and zinc sulfide [ZnS] shell. The shell is surrounded by a dipeptide coating consisting of glycine-histidine, histidine-leucine, carnosine, and amino-PEG (polyethylene glycol) cross-linked via aminobenzophenone and 3-[Tris(hydroxymethyl)phosphonio]propionate (THPP). The dipeptide coating is coordinatively bound via its imidazole rings to the zinc ions of the shell structure. Free primary amino groups are available in the dipeptide coating for coupling reactions. Additional information concerning the dipeptide coating is disclosed in the US patent application US 2003/0059635 A1.

QDBP-655 is sold by Life Technologies Corporation (Eugene, Oregon, USA). It is delivered as a reddish colloidal suspension in buffer. The nanoparticles exhibit the following product characteristics:

| | |
|---|---|
| Appearance: | Reddish clear liquid |
| Hydrodynamic radius: | ≤ 6.5 nm (determined by size exclusion chromatography) |
| Molecular Weight: | 1000 kDa |
| Emission Maximum: | 655 nm ± 4 nm |

### Coupling:

The expressed and affinity-purified peptides A, B or C are dialysed for 2 hours against 50 mM sodium borate pH =8.3 (Slide-A-Lyzer Mini Dialysis Unit - 10 KDa; (Thermo Fisher, Rockford, IL, USA). Afterwards the protein concentration is determined and the solution diluted to the required concentration. The QDBP-655 quantum dot and the protein are mixed together to an end concentration of 0.3µM (QDBP-655) in QDBP-655/ligand ratios of 1:12.5, 1:25, 1:50, 1:75 and 1:100 in sterile borosilicate vials as summarized in the following table:

| Q_CD44v5 | QDBP/L | n_{QDBP-655} | C_{QDBP-655} | V_{QDBP-655} | V_{Ligand} | n_{Ligand} | C_{Ligand} |
|---|---|---|---|---|---|---|---|
| _X1 | 1:- | 78 pmol | 3.9 pmol | 20 µl | 240 µl | - | - |
| _X2 | 1:12.5 | 78 pmol | 3.9 pmol | 20 µl | 240 µl | 975 pmol | 4.06 pmol/µl |
| _X3 | 1:25 | 78 pmol | 3.9 pmol | 20 µl | 240 µl | 1950 pmol | 8.125 pmol/µl |
| _X4 | 1:50 | 78 pmol | 3.9 pmol | 20 µl | 240 µl | 3990 pmol | 16.25 pmol/µl |
| _X5 | 1:75 | 78 pmol | 3.9 pmol | 20 µl | 240 µl | 5850 pmol | 24.4 pmol/µl |
| _X6 | 1:100 | 78 pmol | 3.9 pmol | 20 µl | 240 µl | 7800 pmol | 32.5 pmol/µl |

The successful coupling is shown by SDS-agarose gel-electrophoresis, whereby a ratio of 1:50 or more leads to a dramatic increase in molecular weight showing the successful coupling reaction.

### Immunocytochemical analysis

The immuncytochemical analysis of the tumor cells was performed as follows:
- Each 5 x 10⁵ tumor cells on coating glasses
- Cultivation for 48 hours at 37°C, 5% CO₂
- Washing of the cells for 3 x 1 minute with 1 mL D-PBS
- Fixation for 20 minutes with 1 ml dimethylsuberimidate (DMS) solution (20 mg/ml DMS in 250 ml Tris-HCI, 20 mM CaCl₂, pH 8.0)
- Washing of the cells for 1 x 1 minute with 1 mL D-PBS
- Quenching by incubation for 20 min in 0.2% glycine in D-PBS
- Blocking by incubation for 60 min in 1 % BSA / 5% goat serum in D-PBS
- Incubation with 50µl of the preincubated Qdot-peptide conjugates (100 nM) at RT
- Washing of the cells for 3 x 5 minute with 1 mL D-PBS
- Incubation with 50µl primary antibody (anti-CD44v5: VFF-7, 1:25)
- Washing of the cells for 3 x 5 minute with 1 mL D-PBS
- Incubation with 50µl secondary antibody (GAM-A488, 1:100)
- Washing of the cells for 3 x 5 minute with 1 mL D-PBS
- Conterstaining with 300 µl Hoechst 33342 (200ng/ml in D-PBS) for 10 minutes
- Washing of the cells for 3 x 5 minute with 1 mL D-PBS
- Embedding in Mowiol 4-88 (Sigma Aldrich, St. Louis, MI, USA)

### Results:

The nanoparticles coupled to peptides A, B or C were tested with the human colon carcinoma cell line HCT-116 (see Figure 22). When using the peptide conjugated nanoparticles a labelling of the tumour cells could be observed which colocalizes with the labeling as generated by the anti-CD44v5 antibody as positive control.

## Claims

1. A protein capable of binding to a polypeptide encoded by exon 9 of human CD44 (CD44ex9), said protein comprises or consists of
(i) an amino acid sequence according SEQ ID No. 1 to 5 and SEQ ID No. 39 to 52; or
(ii) an amino acid sequence with at least 85% identity to the amino acid sequence given in SEQ ID No. 1 to 5, SEQ ID No. 39 to 50, and SEQ ID No. 52 as a whole;
(iii) an amino acid sequence with at least 95% identity to the amino acid sequence given in SEQ ID No. 51 as a whole; and
wherein said protein has a length of 100 amino acids or less.

2. The protein capable of binding to CD44ex9 according claim 1, wherein it binds to a CD44 isoform comprising the domain encoded by exon 9, which is preferably selected from the list consisting of:
a) CD44 v5,
b) CD44 v5 -v6,
c) CD44 v3 - v6,
d) CD44 v2 - v10,
e) CD44 v3 - v10,
f) CD44 V4 - v7,
g) CD44 v4 - v10,

3. The protein capable of binding to CD44ex9 according claim 1 or 2 fused to a heterologous protein or heterologous polypeptide, selected from the group consisting of
a) an antibody,
b) a toxin,
c) a cytokine,
d) a protein selected from the group consisting of cytosine deaminase, soluble Fms-like tyrosine kinase ligand, herpes simplex virus-1 thymidine kinase (HSV1-TK), cytochrome P450 2B1, retinoblastoma related proteins, p16/cdkn2 and MMAC1/PTEN, or preferably
e) an enzyme which catalyses the generation of a cytotoxic or cytostatic agent from a precursor molecule or the attachment of a label to the protein.

4. The protein capable of binding to CD44ex9 according claim 1 or 2 conjugated to a heterologous protein or heterologous polypeptide, which is preferably an enzyme which catalyses the generation of a cytotoxic or cytostatic agent from a precursor molecule or the attachment of a label to the protein.

5. A conjugate comprising
a) the protein capable of binding to CD44ex9 according claims 1 to 3;
b) a compound selected from the group consisting of a carbohydrate, a dye molecule, a radioactive isotope, a toxin, a cytostatic agent, a cytokine, a immunomodulatory agent, or a prodrug thereof,
wherein said compound is linked directly or via a linker molecule to the protein capable of binding to CD44ex9.

6. An isolated nucleic acid molecule encoding the protein capable of binding to CD44ex9 according to claims 1 to 3.

7. An expression vector containing the nucleotide sequence according to claim 6.

8. A host cell containing the expression vector according claim 7.

9. A method of producing the protein capable of binding to CD44ex9 of claims 1 to 3, the method comprising:
a) Transforming a host cell with an expression construct comprising a nucleic acid molecule encoding the protein of claims 1 to 3; and
b) Culturing the host cell under conditions suitable for producing the protein capable of binding to CD44ex9 or the respective fusion protein.

10. The protein capable of binding to CD44ex9 as claimed in any one of claims 1 to 3 or of the conjugate as claimed in claim 4 or 5 for the use in the treatment of a disease selected from the group consisting of autoimmune diseases such as insulin-dependent diabetes, multiple sclerosis, Sjögren's syndrome or systemic lupus erythematosus (SLE); skin diseases as psoriasis or atopic dermatitis; chronic inflammatory diseases such as rheumatoid arthritis or inflammatory bowel disease; tissue injury; allergic diseases and cancer disease.

11. Use of the protein capable of binding to CD44ex9 as claimed in any one of claims 1 to 3 or of the conjugate as claimed in claim 4 or 5 for in vitro-diagnosis of a disease selected from the group consisting of autoimmune diseases such as insulin-dependent diabetes, multiple sclerosis, Sjögren's syndrome or systemic lupus erythematosus (SLE); skin diseases as psoriasis or atopic dermatitis; chronic inflammatory diseases such as rheumatoid arthritis or inflammatory bowel disease; tissue injury; allergic diseases and cancer disease.

12. The protein capable of binding to CD44ex9 as claimed in any one of claims 1 to 3 or of the conjugate as claimed in claim 4 or 5 for the use in the in vivo-diagnosis of a disease selected from the group consisting of autoimmune diseases such as insulin-dependent diabetes, multiple sclerosis, Sjögren's syndrome or systemic lupus erythematosus (SLE); skin diseases as psoriasis or atopic dermatitis; chronic inflammatory diseases such as rheumatoid arthritis or inflammatory bowel disease; tissue injury; allergic diseases and cancer disease.

13. The use of the protein capable of binding to CD44ex9 as claimed in any one of claims 1 to 3 or the conjugate according to claim or 5, for in vitro diagnosis, wherein it serves as a diagnostic agent for the discriminating visualization of morphological or functional structures in biological systems.

14. The protein capable of binding to CD44ex9 as claimed in any one of claims 1 to 3 or the conjugate according to claim 4 or 5, for the use in the in vivo-diagnosis, wherein it serves as a diagnostic agent for the discriminating visualization of morphological or functional structures in biological systems in living people, preferably to assist a medical intervention.

15. A pharmaceutical composition comprising the protein capable of binding to CD44ex9 as claimed in any one of claims 1 to 3 or the conjugate according to claim 4 or 5 and a pharmaceutically acceptable excipient.

## Patentansprüche

1. Ein Protein, das an ein von Exon 9 von humanem CD44 (CD44ex9) kodiertes Polypeptid binden kann, wobei das Protein
(i) eine Aminosäuresequenz nach SEQ ID Nr. 1 bis 5 und nach SEQ ID Nr. 39 bis 52; oder
(ii) eine Aminosäuresequenz mit insgesamt mindestens 85 %iger Identität zu der in SEQ ID Nr. 1 bis 5, SEQ ID Nr. 39 bis 50 und SEQ ID Nr. 52 angegebenen Aminosäuresequenz;
(iii) eine Aminosäuresequenz mit insgesamt mindestens 95 %iger Identität zu der in SEQ ID Nr. 51 angegebenen Aminosäuresequenz; umfasst oder daraus besteht,
wobei das Protein eine Länge von 100 Aminosäuren oder weniger aufweist.

2. Protein mit Bindungsmöglichkeit an CD44ex9 nach Anspruch 1, wobei es an eine CD44 Isoform bindet, die die von dem Exon 9 kodierte Domäne umfasst, welche bevorzugt aus der Liste ausgewählt ist, die aus:
a) CD44 v5,
b) CD44 v5 - v6,
c) CD44 v3 - v6,
d) CD44 v2 - v10
e) CD44 v3 - v10,
f) CD44 V4 - v7,
g) CD44 v4 - v10,
besteht.

3. Das Protein mit Bindungsmöglichkeit an CD44ex9 nach Anspruch 1 oder 2, fusioniert mit einem heterologen Protein oder einem heterologen Polypeptid, das ausgewählt ist aus der Gruppe bestehend aus:
a. Einem Antikörper,
b. Einem Toxin,
c. Einem Zytokin,
d. Einem Protein ausgewählt aus der Gruppe bestehend aus Cytosindeaminase, löslicher Fms-ähnlicher Tyrosinkinaseligand, Herpes simplex virus-1 Thymidinkinase (HSV1-TK), Cytochrom P450 2B1, Retinoblastomproteine, p16/dcdkn2 und MMAC1/PTEN; oder bevorzugt
e. Ein Enzym, das die die Bildung einer zytotoxischen oder zytostatischen Substanz ausgehend von einem Vorläufermolekül katalysiert oder die Anbindung eines Markers an das Protein katalysiert.

4. Das Protein mit Bindungsmöglichkeit an CD44ex9 nach Anspruch 1 oder 2, konjugiert mit einem heterologen Protein oder einem heterologen Polypeptid, welches bevorzugt ein Enzym ist, das die die Bildung einer zytotoxischen oder zytostatischen Substanz ausgehend von einem Vorläufermolekül katalysiert oder die Anbindung eines Markers an das Protein katalysiert.

5. Ein Konjugat umfassend:
a. Das Protein gemäß einem der Ansprüche 1 bis 3;
b. Eine Verbindung ausgewählt aus der Gruppe bestehend aus einem Kohlenhydrat, einem Farbstoffmolekül, einem radioaktiven Isotop, einem Toxin, einem zytostatischen Agens, einem Zytokin, einem immunmodulatorischen Agens, oder einem Vorläufermolekül hiervon,
wobei diese Verbindung direkt oder über ein Linkermolekül an das Protein, das an CD44ex9 binden kann, verknüpft ist.

6. Ein isoliertes Nukleinsäuremolekül, das für das Protein mit Bindungsmöglichkeit an CD44ex9 gemäß einem der Ansprüche 1 bis 3 kodiert.

7. Ein Expressionsvektor, umfassend die Nukleotidsequenz gemäß Anspruch 6.

8. Eine Wirtszelle aufweisend den Expressionsvektor gemäß Anspruch 7.

9. Ein Verfahren zur Herstellung eines Proteins mit Bindungsmöglichkeit an CD44ex9 umfassend:
a. Transformation einer Wirtszelle mit einem Expressionskonstrukt umfassend ein Nukleinsäuremolekül, das für das Protein gemäß einem der Ansprüche 1 bis 3 kodiert.
b. Kultivieren der Wirtszelle unter Bedingungen, die die Herstellung des Proteins mit Bindungsmöglichkeit an CD44ex9 oder dem entsprechenden Fusionsprotein ermöglichen.

10. Protein mit Bindungsmöglichkeit an CD44ex9 nach einem der Ansprüche 1 bis 3 oder das Konjugat nach Anspruch 4 oder 5 zur Anwendung bei der Behandlung einer Erkrankung ausgewählt aus der Gruppe bestehend aus Autoimmunerkrankungen, wie Insulin-pflichtiger Diabetes, Multiple Sklerose, Sjögren'sches Syndrom oder systemischer Lupus erythematodes (SLE); Hauterkrankungen wie Psoriasis oder atopische Dermatitis; chronisch-entzündliche Erkrankungen wie rheumatoide Arthritis oder entzündliche Darmerkrankungen; Gewebeverletzungen; allergische Erkrankungen und Krebserkrankungen.

11. Verwendung eines Proteins mit Bindungsmöglichkeit an CD44ex9 nach einem der Ansprüche 1 bis 3 oder eines Konjugats nach Anspruch 4 oder 5 für die *in vitro*-Diagnose einer Erkrankung ausgewählt aus der Gruppe bestehend aus Autoimmunerkrankungen, wie Insulin-pflichtiger Diabetes, Multiple Sklerose, Sjögren'sches Syndrom oder systemischer Lupus erythematodes (SLE); Hauterkrankungen wie Psoriasis oder atopische Dermatitis; chronisch-entzündliche Erkrankungen wie rheumatoide Arthritis oder entzündliche Darmerkrankungen; Gewebeverletzungen; allergische Erkrankungen und Krebserkrankungen.

12. Verwendung eines Proteins mit Bindungsmöglichkeit an CD44ex9 nach einem der Ansprüche 1 bis 3 oder eines Konjugats nach Anspruch 4 oder 5 für die *in vivo*-Diagnose einer Erkrankung ausgewählt aus der Gruppe bestehend aus Autoimmunerkrankungen, wie Insulin-pflichtiger Diabetes, Multiple Sklerose, Sjögren'sches Syndrom oder systemischer Lupus erythematodes (SLE); Hauterkrankungen wie Psoriasis oder atopische Dermatitis; chronisch-entzündliche Erkrankungen wie rheumatoide Arthritis oder entzündliche Darmerkrankungen; Gewebeverletzungen; allergische Erkrankungen und Krebserkrankungen.

13. Verwendung eines Proteins mit Bindungsmöglichkeit an CD44ex9 nach einem der Ansprüche 1 bis 3 oder eines Konjugats nach Anspruch 4 oder 5 für die in vitro Diagnose, wobei es als diagnostisches Agens für die diskriminierende Visualisierung morphologischer und funktionaler Strukturen in biologischen Systemen dienen kann.

14. Verwendung eines Proteins mit Bindungsmöglichkeit an CD44ex9 nach einem der Ansprüche 1 bis 3 oder eines Konjugats nach Anspruch 4 oder 5 für die in vivo Diagnose, wobei es als diagnostisches Agens für die diskriminierende Visualisierung morphologischer und funktionaler Strukturen in biologischen Systemen in lebenden Menschen dienen kann, bevorzugt um eine medizinische Intervention zu unterstützen.

15. Eine pharmazeutische Zusammensetzung umfassend das konjugierte Nanopartikel nach einem der Ansprüche 1 bis 3 oder das Konjugat nach Anspruch 4 oder 5 und einen pharmazeutisch verträglichen Hilfsstoff.

## Revendications

1. Protéine capable de se fixer à un polypeptide codé par l'exon 9 de CD44 humain (CD44ex9), ladite protéine comprend ou est constituée de :
(i) une séquence d'acides aminés selon SEQ ID N° 1 à 5 et SEQ ID N°39 à 52 ; ou
(ii) une séquence d'acides aminés avec au moins 85 % d'identité à la séquence d'acides aminés donnée dans SEQ ID N° 1 à 5, SEQ ID N° 39 à 50 et SEQ ID N° 52 dans l'ensemble ;
(iii) une séquence d'acides aminés avec au moins 95 % d'identité à la séquence d'acides aminés donnée dans SEQ ID N° 51 dans l'ensemble ; et
dans laquelle ladite protéine a une longueur de 100 acides aminés ou moins.

2. Protéine capable de se fixer à CD44ex9 selon la revendication 1, dans laquelle elle se fixe à une isoforme de CD44 comprenant le domaine codé par l'exon 9, de préférence sélectionnée dans la liste constituée de :
a) CD44 v5,
b) CD44 v5-v6,
c) CD44 v3-v6,
d) CD44 v2-v10,
e) CD44 v3-v10,
f) CD44 V4-v7,
g) CD44 v4-v10.

3. Protéine capable de se fixer à CD44ex9 selon la revendication 1 ou 2 fusionnée à une protéine hétérologue ou un polypeptide hétérologue, sélectionné(e) dans le groupe constitué de
a) un anticorps,
b) une toxine,
c) une cytokine,
d) une protéine sélectionnée dans le groupe constitué de cytosine désaminase, d'un ligand de tyrosine kinase analogue à Fms soluble, de thymidine kinase du virus de l'herpes simplex (HSV1-TK), du cytochrome P450 2B1, des protéines rétinoblastomes p16/cdkn2 et MMAC1/PTEN, ou de préférence
e) une enzyme qui catalyse la génération d'un agent cytotoxique ou cytostatique par une molécule précurseur ou le marquage de la protéine.

4. Protéine capable de se fixer à CD44ex9 selon la revendication 1 ou 2 conjuguée à une protéine hétérologue ou un polypeptide hétérologue, de préférence une enzyme qui catalyse la génération d'un agent cytotoxique ou cytostatique par une molécule précurseur ou le marquage de la protéine.

5. Un conjugué composé de
a) la protéine capable de se fixer à CD44ex9 selon l'une des revendications 1 à 3;
b) un composé sélectionné dans le groupe constitué d'un glucide, d'une molécule colorante, d'un isotope radioactif, d'une toxine, d'un agent cytostatique, d'une cytokine d'un agent immunomodulateur ou d'un promédicament de celui-ci,
où ledit composé est relié directement ou par une molécule de liaison à la protéine capable de se fixer à CD44ex9.

6. Molécule d'acide nucléique isolée codant la protéine capable de se fixer à CD44ex9 selon l'une des revendications 1 à 3.

7. Vecteur d'expression contenant la séquence nucléotidique selon la revendication 6.

8. Cellule hôte contenant le vecteur d'expression selon la revendication 7.

9. Méthode de production de la protéine capable de se fixer à CD44ex9 selon les revendications 1 à 3, ladite méthode consistant à :
a) Transformer une cellule hôte à l'aide d'une structure d'expression comprenant une molécule d'acide nucléique codant la protéine des revendications 1 à 3 ; et
b) Cultiver la cellule hôte dans des conditions adéquates pour produire la protéine capable de se fixer à CD44ex9 ou la protéine de fusion correspondante.

10. Protéine capable de se fixer à CD44ex9 selon l'une des revendications 1 à 3 ou du conjugué selon la revendication 4 ou 5 destinée à être utilisée dans le traitement d'une maladie sélectionnée parmi le groupe constitué de maladies auto-immunes telles que le diabète insulinodépendant, la sclérose en plaques, le syndrome de Sjögren ou le lupus érythémateux systémique (SLE) ; de maladies cutanées telles que le psoriasis ou la dermatite atopique ; de maladies inflammatoires chroniques telles que la polyarthrite rhumatoïde ou la maladie du côlon inflammatoire ; d'une lésion tissulaire ; de maladies allergiques et d'une maladie cancéreuse.

11. Utilisation de la protéine capable de se fixer à CD44ex9 selon l'une des revendications 1 à 3 ou du conjugué selon la revendication 4 ou 5 pour établir un diagnostic in vitro d'une maladie sélectionnée parmi le groupe constitué de maladies auto-immunes telles que le diabète insulinodépendant, la sclérose en plaques, le syndrome de Sjögren ou le lupus érythémateux systémique (SLE) ; de maladies cutanées telles que le psoriasis ou la dermatite atopique ; de maladies inflammatoires chroniques telles que la polyarthrite rhumatoïde ou la maladie du côlon inflammatoire ; d'une lésion tissulaire ; de maladies allergiques et d'une maladie cancéreuse.

12. Protéine capable de se fixer à CD44ex9 selon l'une des revendications 1 à 3 ou du conjugué selon la revendication 4 ou 5 destinée à être utilisée dans le diagnostic in vivo d'une maladie sélectionnée parmi le groupe constitué de maladies auto-immunes telles que le diabète insulinodépendant, la sclérose en plaques, le syndrome de Sjögren ou le lupus érythémateux systémique (SLE) ; de maladies cutanées telles que le psoriasis ou la dermatite atopique ; de maladies inflammatoires chroniques telles que la polyarthrite rhumatoïde ou la maladie du côlon inflammatoire ; d'une lésion tissulaire ; de maladies allergiques et d'une maladie cancéreuse.

13. Utilisation de la protéine capable de se fixer à CD44ex9 selon l'une des revendications 1 à 3 ou du conjugué selon la revendication 4 ou 5 pour établir un diagnostic in vitro, où elle agit en qualité d'agent de diagnostic permettant la visualisation discriminante des structures morphologiques et fonctionnelles des systèmes biologiques.

14. Protéine capable de se fixer à CD44ex9 selon l'une des revendications 1 à 3 ou du conjugué selon la revendication 4 ou 5 destinée à un utilisation dans le diagnostic in vivo, où elle agit en qualité d'agent de diagnostic permettant la visualisation discriminante des structures morphologiques et fonctionnelles des systèmes biologiques de personnes vivantes, de préférence pour assister une intervention médicale.

15. Composition pharmaceutique comprenant la protéine capable de se fixer à CD44ex9 selon l'une des revendications 1 à 3 ou le conjugué selon la revendication 4 ou 5 ainsi qu'un excipient pharmaceutiquement acceptable.
